(19) Europäisches Patentamt
European Patent Office
Office européen des brevets

(11) Publication number: **0 275 131 B1**

(12) # EUROPEAN PATENT SPECIFICATION

(45) Date of publication of patent specification: **23.09.92**

(51) Int. Cl.[5]: **C07D 209/48**, C07D 405/12, A01N 37/32

(21) Application number: **88200053.2**

(22) Date of filing: **13.01.88**

(54) **Herbicidal oxoindanes and tetrahydronaphthalenes.**

(30) Priority: **14.01.87 US 3232**

(43) Date of publication of application: **20.07.88 Bulletin 88/29**

(45) Publication of the grant of the patent: **23.09.92 Bulletin 92/39**

(84) Designated Contracting States: **AT BE CH DE ES FR GB GR IT LI LU NL SE**

(56) References cited:
**EP-A- 0 104 484**
**EP-A- 0 170 191**

**CHEMICAL ABSTRACTS, vol. 63, no. 9, October 25, 1965, Columbus, Ohio, USA - Herbert O. HOUSE et al. :"By-Products of the Robinson annelation reaction with cyclohexanone, cyclopentanone, and cyclopentane-1,2-dione", abstract no. 11477c**

**CHEMICAL ABSTRACTS, vol. 105, no. 19, November 10, 1986, Columbus, Ohio, USA - SIRCAR, ILA et al.:"Cardiotonic agents. 4. Synthesis and biological evaluation on N-substituted 2,4,4a,5-tetrahydro-3H-indeno[1,2-c]pyridazin-3-ones : rigid structures derived from C I - 930 and analogs", page 740, column 2, abstract no. 172382d**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Semple, Joseph Edward**
**436 Coldspring Run**
**Newark, DE 19711(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to certain novel (N-heterocyclic)-substituted oxoindanes and tetrahydronaphthalenes, their preparation, herbicidal compositions comprising these compounds and their use as herbicides.

European Patent Application Publication No. 170191 discloses herbicidal compounds of the following formula:

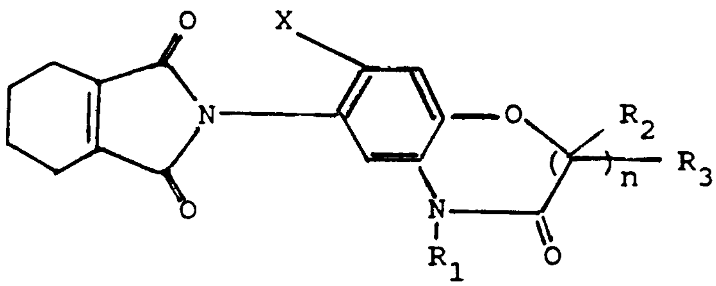

where

X represents a hydrogen, chlorine or fluorine atom;

n is 0 or 1;

$R_1$ represents a hydrogen atom or an alkyl, alkenyl, haloalkyl, haloalkenyl, haloalkynyl, alkoxyalkyl, or alkoxyalkoxyalkyl group; and

$R_2$ and $R_3$ represent a hydrogen or halogen atom or an alkyl or phenyl group.

Japanese patent publication No.61 194 072 discloses intermediates useful to prepare herbicidal compounds of the formula

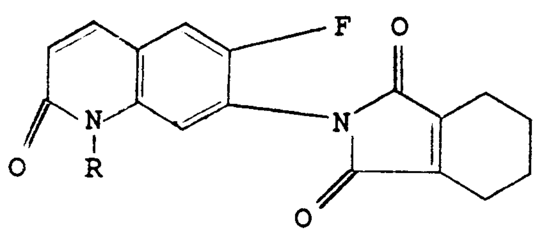

The presence of undesired vegetation causes substantial damage to useful crops, especially agricultural products that satisfy man's basic food needs, such as soybeans, barley and wheat. The current population explosion and concomitant world food shortage demand improvements in the efficiency of producing these crops. Prevention or minimizing the lose of a portion of valuable crops by killing, or inhibiting the growth of undesired vegetation is one way of improving this efficiency.

A wide variety of materials useful for killing, or inhibiting (controlling) the growth of undesired vegetation is available; such materials are commonly referred to as herbicides. The need exists, however, for still more effective herbicides that destroy or retard weeds without causing significant damage to useful crops.

It has been found that useful phytotoxic properties are possessed by 6-(heterocyclic)-1-oxoindanes and 7-(heterocyclic)-1-oxo-1,2,3,4-tetra-hydronaphthalenes of the formula:

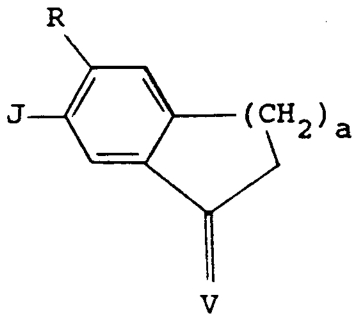

(I)

wherein

R is a hydrogen or halogen atom; a cyano group; an alkyl or alkoxy group having one to four carbon atoms; a trihalomethyl or hydroxymethyl group; an alkyl-, alkenyl-, or alkanoyl-oxymethyl group of up to six carbon atoms; or a group $-OCF_nH_{3-n}$ wherein n = one, two or three;

V is an oxygen atom or a group N-O-$R^1$, wherein $R^1$ represents a hydrogen atom; an alkyl, alkenyl or alkynyl group having up to six carbon atoms, the alkyl group optionally being substituted by cyano, mono- or poly- halo, or hydroxy- or alkoxy-carbonyl and the alkenyl group being optionally mono- or poly- halo-substituted; a phenyl group optionally substituted by one or more groups $R^{14}$ as defined below or a phenylalkyl group having seven to ten carbon atoms optionally substituted with one or more groups $R^{14}$ as defined below; or a heteroaryl or heteroaralkyl group wherein the heteroaryl moiety is furanyl, pyrrolyl, thienyl, pyridinyl, thiazolyl, isoxazolyl, pyrazolyl, imidazolyl, triazolyl (1,2,3- or 1,2,4-), benzofuranyl, indolyl, indazolyl, thianaphthenyl, pyridazinyl, pyrimidinyl, pyrazinyl, cinnolinyl, quinazolinyl or quinoxalinyl;

a is one or two

J is one of the moieties:

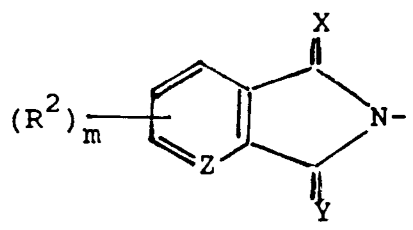

J-1

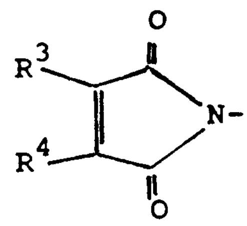

J-2

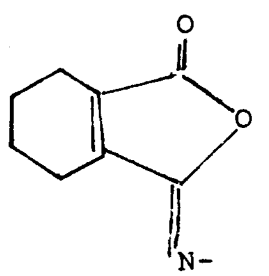

J-3

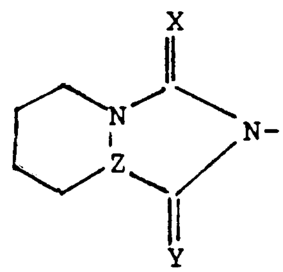

J-4

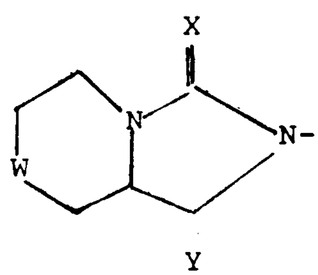

J-5

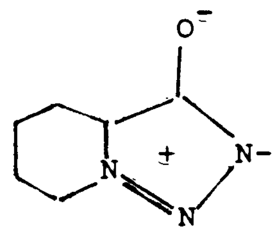

J-6

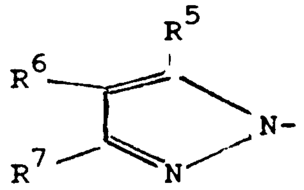

J-7

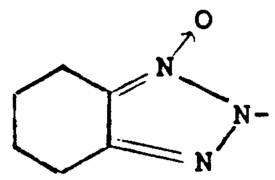

J-8

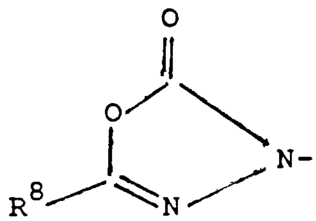

J-9

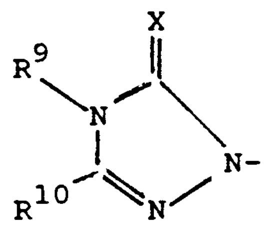

J-10

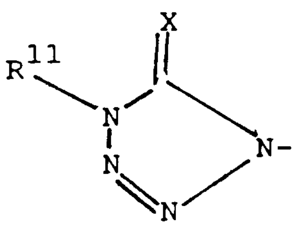

J-11

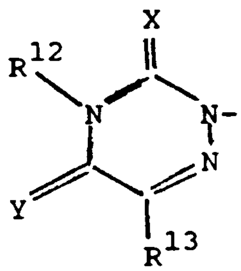

J-12

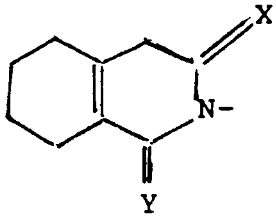

J-13

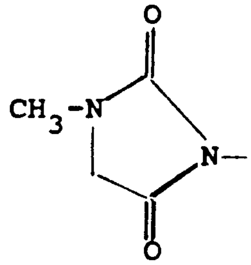

J-14

wherein

X and Y each independently represents 0 or S;
Z is CH or N;
W is S or $SO_2$;
m is zero, one or two;
$R^2$ is a halogen atom, or is an alkyl, haloalkyl or alkoxy group having one to four carbon atoms;
$R^3$ and $R^4$ each independently represents a hydrogen atom or an alkyl group having one to four carbon atoms, or $R^3$ and $R^4$ together with the carbon atoms to which they are attached form an alicyclic six-membered ring that may be substituted by one to three methyl group;
$R^5$ is a hydrogen or halogen atom, or an alkyl or alkyl-$S(O)_b$- group having one to four carbon atoms wherein b is zero, one or two;
$R^6$ is a hydrogen or halogen atom, or an alkyl, alkylthio or alkylsulfonyl group having one to four carbon atoms;
$R^7$ is an alkyl group having one to four carbon atoms, or $R^6$ and $R^7$ together form the moiety, $-(CH_2)_c-$, wherein c is three or four, which may be substituted by one to three methyl group;
$R^8$ is a hydrogen atom or an alkyl, alkenyl, haloalkyl or alkoxyalkyl group having up to six carbon atoms, or a cycloalkyl group having three to six carbon atoms, a cycloalkylalkyl group having four to eight carbon atoms, or either of these groups substituted by one to three alkyl groups each having one to four carbon atoms;
$R^9$ has one to six carbon atoms and is an alkyl, haloalkyl, cyanoalkyl, alkoxyalkyl, alkenyl, alkynyl, or -alkyl-$S(O)_a$-alkyl group where a is zero, one or two;
$R^{10}$ is a hydrogen atom, a group $R^9$ as defined above, or $R^9$ and $R^{10}$ together represent an alkylene moiety $-(CH_2)_c-$, as defined above for $R^7$, or a moiety $-(CH_2)_d-U-(CH_2)_e-$ wherein d and e each is zero, one, two or three, and d plus e = two or three, and U is oxygen, $-S(O)_a-$ or -NRalkyl where a is zero, one or two with the proviso that when U is $-S(O)_a-$, d is other than zero;
$R^{11}$ has one to six carbon atoms, and is an alkyl, haloalkyl, alkoxyalkyl, alkylthioalkyl, cyanoalkyl, haloalkoxyalkyl, alkenyl or haloalkenyl group;
$R^{12}$ is a hydrogen atom, or an amino group, or has one to four carbon atoms and is an alkyl, haloalkyl, cyanoalkyl, alkenyl, alkynyl, alkoxyalkyl, or alkoxycarbonyl group.
$R^{13}$ is a group $R^{11}$ as defined above, or is a hydrogen atom, or a carboxyl or alkoxycarbonyl group;
$R^{14}$ is a halogen atom, or a $C_1$ to $C_3$ alkyl, $C_1$ to $C_3$ alkoxy, $CF_3$, $NO_2$, CN or $OCF_2H$ group;
and salts of the acidic species with alkali metal bases, ammonia and amines.

In these compounds, each aliphatic group can be either straight chain or branched-chain. "Halogen" is bromine, chlorine and fluorine. Suitable amine salts are those of mono-, di- and tri-alkyl- and -alkanol-amines wherein each alkyl moiety contains from one to twenty carbon atoms.

Preferred for reasons of increased ease of synthesis and/or greater phytotoxic properties are:

1. Compounds of Formula I where
R is H, halogen or $C_1$ to $C_3$ alkyl;
$R^1$ is H, $C_1$ to $C_6$ alkyl, $C_3$ to $C_5$ alkenyl, $C_3$ to $C_5$ alkynyl, $C_3$ to $C_8$ alkoxycarbonylalkyl, or benzyl; and
$R^{14}$ is F, Cl, $CH_3$ or $OCH_3$.

2. Compounds of preferred group 1 above where a is one.

3. Compounds of preferred group 1 above where a is two.

4. Compounds of preferred group 1 above where
$R^2$ is F, Cl, $CH_3$ or $OCH_3$;
m is 0 or 1; R is H, F, Cl or $C_1$ to $C_3$ alkyl;
$R^1$ is H, $C_1$ to $C_4$ alkyl, allyl, propargyl, optionally substituted benzyl, or $C_3$ to $C_8$ alkoxycarbonylalkyl.

5. Compounds of preferred group 4 above where
m is O;
V is $=NOR^1$;
X and Y are O; and
R is H, F, Cl or $CH_3$.

6. Compounds of preferred group 5 above where
$R^3$ and $R^4$ form an alicyclic six-membered ring;
R is H or $CH_3$;
$R^1$ is H, $C_1$ to $C_4$ alkyl, allyl, propargyl, optionally substituted benzyl, or $C_3$ to $C_8$ alkoxycarbonylalkyl.

7. Compounds of preferred group 6 above where
R is H or F; and
$R^3$ and $R^4$ form $-(CH_2)_4-$.

8. Compounds of preferred group 1 above where J is J-1.

9. Compounds of preferred group 1 above where J is J-2.
10. Compounds of preferred group 1 above where J is J-3.
11. Compounds of preferred group 1 above where J is J-4.
12. Compounds of preferred group 1 above where J is J-5.
13. Compounds of preferred group 1 above where J is J-6.
14. Compounds of preferred group 1 above where J is J-7.
15. Compounds of preferred group 1 above where J is J-8.
16. Compounds of preferred group 1 above where J is J-9.
17. Compounds of preferred group 1 above where J is J-10.
18. Compounds of preferred group 1 above where J is J-11.
19. Compounds of preferred group 1 above where J is J-12.
20. Compounds of preferred group 1 above where J is J-13.
21. Compounds of preferred group 1 above where J is J-14.
22. Compounds of preferred group 14 above where
$R^5$ is H, Cl, $CH_3$, $SCH_3$ or $SO_2CH_3$;
$R^6$ is H, Cl, $CH_3$, $SCH_3$ or $SO_2CH_3$;
$R^7$ is $C_1$ to $C_2$ alkyl; and
$R^6$ and $R^7$ taken together are $(CH_2)_3$ or $-(CH_2)_4$.
23. Compounds of preferred group 16 above where
$R^8$ is H, $C_1$ to $C_4$ alkyl or $C_3$ to $C_6$ cycloalkyl.
24. Compounds of preferred group 17 above where
$R^9$ is $C_1$ to $C_3$ alkyl, allyl, propargyl, or $C_1$ to $C_3$ haloalkyl;
X is O;
$R^{10}$ is H or $C_1$ to $C_3$ alkyl; and
$R^9$ and $R^{10}$ taken together form $(CH_2)_3$, $-(CH_2)_4$ or $-CH_2SCH_2-$.
25. Compounds of preferred group 18 above where
X is O; and
$R^{11}$ is $C_1$ to $C_3$ alkyl, $CH_2CN$,
$C_1$ to $C_3$ haloalkyl.
26. Compounds of preferred group 19 above where
X and Y are 0;
$R^{12}$ is H, $NH_2$, $C_1$ to $C_3$ alkyl, allyl or propargyl; and
$R^{13}$ is H, $C_1$ to $C_3$ alkyl, $CH_2CN$, $CO_2H$ or $CO_2CH_3$.
27. Compounds of preferred group 2 above where J is J-1, J-2, J-7, J-10 or J-12.
28. Compounds of preferred group 3 above where
J is J-1, J-2, J-7, J-10 or J-12.

The compounds of the invention can exist as isomers, e.g., when X is N-O-$R^1$. The synand anti isomers have been found to be both active. The present invention contemplates all herbicidally effective stereoisomers and stereoisomeric mixtures resulting from the manner of preparation, or deliberately created.

Non-limiting representative species of the invention include
N-(5-chloro-1-oxo-6-indanyl)-3,4,5,6-tetrahydrophthalimide,
N-(1-(ethoxyimino)-1,2,3,4-tetrahydro-7-naphthalenyl)-3,4,5,6-tetrahydrophthalimide,
N-((1-((hydroxycarbonyl)methoxy)imino)-1,2,3,4-tetrahydro-7-naphthalenyl)-3,4,5,6-tetrahydrophthalimide.
N-(5-methyl-1-(isopropoxyimino)-6-indanyl)-3,4,-5,6-tetrahydrophthalimide,
N-(5-fluoro-1-(cyclobutylmethoxyimino)-6-indanyl)-3,4,5,6-tetrahydrophthalimide.
N-(5-fluoro-1-(hydroxycarbonylmethoxyimino)-6-indanyl)-3,4,5,6-tetrahydrophthalimide.
N-(5-fluoro-1-(ethoxycarbonylethoxyimino)-6-indanyl)-3,4,5,6-tetrahydrophthalimide,
and the corresponding compounds wherein J is a group selected from J-1 and J-3 through J-14.

Compounds of Formula I may be prepared by treating an aniline or isocyanate of formula II:

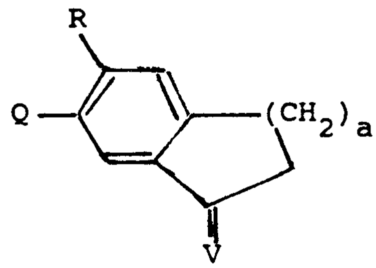

(II)

wherein Q is $-NH_2$ or -NCO with the appropriate reagents to introduce any one of the groups J-1 to J-14 and, optionally, oximating a compound of formula I where V is an oxygen atom to a compound of Formula I where V is the group N-O-$R^1$. These reactions may be conducted as follows:

J = J-1, J-2 (Z = carbon or $R^3$ and $R^4$ complete a ring): these compounds can be prepared by treating an appropriate phthalic, tetrahydrophthalic or maleic anhydride with an appropriate aniline, by fusing the two reagents at a temperature of 120-220°C, or by refluxing a mixture of the reagents in a solvent such as acetic acid, toluene or xylene. The reaction can be catalyzed in xylene or toluene by an amine such as triethylamine or DBU (1,8-diazabicyclo[5.4.0]undec-7-ene). The products wherein one or both of X and Y is (are) sulfur can be prepared by treating+ (i.e., thionating) a product wherein X and Y are both oxygen with phosphorus pentasulfide or Lawesson's Reagent (2,4-bis-(4-methoxyphenyl)-1,3-dithia-2,4-diphosphetane-2,4-disulfide), in an inert solvent such as benzene, toluene, xylene or chloroform at room temperature to reflux temperature. Where Z = nitrogen, an appropriate quinolinic anhydride is treated with an appropriate aniline in two stpes:

(a) stirring the reagents in an inert solvent such as methylene chloride or tetrahydrofuran at room temperature to reflux temperature, to give the intermediate amide/acid, then (b) dehydrating the intermediate with acetic anhydride at 80°C to reflux temperature. Alternatively, the two reagents are stirred at room temperature in toluene to form the intermediate, which then is treated with 0.1 to 0.5 equivalent of triethylamine as catalyst, followed by azeotropic distillation to yield the product. References: U.S. Patents 4,406,690; 4,439,229; U.K. patent 2,150,929;

J = J-3: such are prepared by the method described above for preparing the J-1 and J-2 compounds wherein Z = nitrogen, except that the amide/acid intermediate is dehydrated by using DCC (1,3-dicyclohexylcarbodiimide) or like dehydrating/-condensation agent. Reference: U.S. Patent 4,472,190.

J = J-4, Z = nitrogen, X = O, Y = O or S: 1-(ethoxycarbonyl)piperidazine is treated with an iso(thio)-cyanate prepared from an appropriate aniline by conventional methods, and treating the resulting intermediate product with sodium hydride. Alternatively, an appropriate N-(alkoxycarbonyl)-hydrazine is treated with an appropriate iso(thio)cyanate, and the resulting intermediate is cyclized with a base to provide a urazole. Alkylation of the disodium salt of the urazole with 1,4-dibromobutane yields the desired product. Reference: European Patent 104484; German Offenlegungschrift 3,504,051. J-4 compounds wherein Z = carbon, X = O or S, Y = O: ethyl pipecolinate is treated with the iso(thio)cyanate prepared from the appropriate aniline, in an inert solvent, such as THF, and the resulting (thio)urea intermediate is treated wtih ethanolic hydrogen chloride at reflux. Reference: U.S. Patent 4,560,752. Thionation as described for J = J-1, provides those compounds wherein both X and Y = S.

J = J-5: these are prepared by methods analogous for those described for J = J-4, Z = nitrogen except that the (thio)urea intermediate is treated with either aqueous hydrochloric acid or sodium methoxide in methanol. Reference: European Patent 70,389.

J = J-6: an appropriate aniline is diazotized and coupled with pipecolinic acid in the presence of triethylamine. Reference: U.S. Patents 4,599,104; 4,002,636; 3,939,174.

J = J-7: an appropriate aniline is diazotized and the product is reduced with stannous chloride to give a phenylhydrazine. The phenylhydrazine is treated with a 2-(alkoxycarbonyl)cyclohexanone under dehydrating conditions to yield the hexahydroindazol-3-one, which is chlorinated, optionally in the presence of a dehydrohalogenating agent to provide the J-7 compound wherein $R^5$ is halogen. Reference: European Patent 152,890. Preparation of such compounds, and those wherein $R^5$ is other than halogen is described in European patent 138,527. These patents also teach methods for preparing J-7 compounds wherein $R^6$ and $R^7$ differ from species to species.

J = J-8: an appropriate aniline is converted to a phenylhydrazine, which is treated with 6-(hydroxyimino)-1-morpholinocyclohexene, and the resulting product is oxidized with cupric sulfate in pyridine. Reference: European Patent 142,769.

J = J-9: an appropriate aniline is converted to a phenylhydrazine, which is treated with an acyl halide

to form the acylhydrazide, which is treated with trichloromethyl chloroformate or phosgene, optionally in the presence of triethylamine.

J = J-10: an appropriate aniline is converted to a phenylhydrazine, which is condensed with an alpha-ketoacid to form a hydrazone. The hydrazone is treated with diphenylphosphoryl azide in the presence of triethylamine to give a J-10 compound wherein X = O, $R^9$ = hydrogen, which can be thionated to X = S with phosphorus pentasulfide. Such compounds can be alkylated by conventional procedures to form those wherein $R^9$ = (halo)alkyl. References: U.S. Patents 4,213,773; 4,315,767; WIPO Patent WO 85/01637.

J = J-11: an iso(thio)cyanate prepared from the appropriate aniline is treated with trimethylsilyl azide ($R^{11}$) = hydrogen), which may be alkylated by conventional means: Reference: WIPO Patent WO 85/01939.

J = J-12: an appropriate aniline is diazotized and treated with malonyldiurethane. The resulting product is cyclized by treatment first with ethanolic potassium hydroxide in THF, then with aqueous hydrochloric acid to give a triazinedionecarboxylic acid, which is decarboxylated in the presence of mercaptoacetic acid and xylene to give J-12 wherein $R^{12}$ = hydrogen, which may be converted to $R^{12}$ = one of the defined moieties other than hydrogen. Alternatively, an appropriately substituted aniline is treated with sodium nitrite and tin (II) chloride in aqueous hydrochloric acid to produce the corresponding phenylhydrazine, which is converted to the hydrazone by treatment with acetone in sulfuric acid and THF. Treatment of the hydrazone with potassium cyanate in aqueous acetic acid gives a triazolidinone, which upon treatment with $R^{13}COCO_2H$ and sulfuric acid in dioxane gives J-12 wherein $R^{12}$ = hydrogen. Reference: WIPO Patent WO 86/00072. The patent also describes other suitable methods.

J = J-13: these are prepared by treating tetrahydrohomophthalic acid (2-carboxycyclohexene-1-acetic acid: (R. Grewe and A. Mondon, Chemische Berichte, volume 81, pages 279-286 (1948)), at page 283) with an appropriate aniline in refluxing acetic acid solvent.

J = J-14: an isocyanate prepared from an appropriate aniline is treated with sarcosine methyl ester hydrochloride in the presence of triethylamine, followed by treatment of the resulting urea with aqueous ethanolic hydrogen chloride. Reference: U.S. Patent 4,560,752.

An appropriate aniline or isocyanate reagent can be prepared from a compound of Formula I, or a compound of Formula I wherein J is other than one of those defined herein, prepared by methods described herein. In such a case, the moiety J might appropriately be one of benzhydrlamino, benzoylamino, acetylamino, benzylamino, allylamino and trichloroacetylamino. As is shown in the examples, the aniline or isocyanate can be prepared from the J-compound by conventional means.

Alternatively to the treatment of an aniline or isocyanate of Formula II with a J-reagent, compounds of the invention wherein V is =N-O-$R^1$, particularly those wherein $R^1$ is hydrogen, can be prepared from the corresponding compounds of Formula I wherein V is =O, by treating it with hydroxylamine hydrochloride, or appropriate $R^1$ derivative thereof, suitably by refluxing a mixture of the reagents with sodium acetate in a solvent such as ethanol, or by use of pyridine as solvent at ambient temperature.

Anilines of Formula II -- i.e., 6-amino-1-indanones, and 7-amino-1-tetralones may be prepared by conventional nitration of 1-indanone, preferably substituted by fluorine in the position para to the oxo group, or 1-tetralone, with nitric acid at a low temperature, such as 1-10 °C. The resulting 6-nitro-1-indanone or 7-nitro-1-tetralone is reduced, e.g. by treatment with aqueous sodium hypophosphite in ethanol and in the presence of a catalyst, such as palladium on carbon, or by treating with stannous chloride in ethyl acetate. Corresponding isocyanates can be prepared by treating an aniline of Formula II with phosgene in an inert solvent such as 1,4-dioxane or toluene, initially at room temperature or below, then at reflux.

Compounds of Formula I have been found to affect adversely the growth of some plants, many of which are commonly considered as weeds, and therefore are useful for controlling the growth of such unwanted plants. Some of the compounds of Formula I have been found to have selectivity with respect to some crop plants such as wheat, soybean, rice and grain sorghum, i.e., they control weeds at dosages at which they do not significantly harm the crop plants. They appear to be most effective when applied postemergence (applied to the foliage of the growing plant).

Accordingly, the invention includes a method of combatting unwanted plants which comprises applying to the locus an effective amount of a compound of Formula I. In the cases where is is desired to control weeds in crop plantings, it is of course preferable to employ the lowest dosage that will control the weeds, for this will minimize any possible deleterious effect of the compound upon the crop plants.

For application, a compound of Formula I ordinarily is applied most effectively by formulating it with a suitable inert carrier or surface-active agent, or both. The invention, therefore, also includes compositions suitable for combatting unwanted plants, such compositions comprising an inert carrier or surface-active agent, or both, and as active ingredient at least one compound of Formula I.

The term "carrier" as used herein means an inert solid or liquid material, which may be inorganic or organic and of synthetic or natural origin, with which the active compound is mixed or formulated to

facilitate its application to the plant, seed, soil or other object to be treated, or its storage, transport and/or handling. Any of the materials customarily employed in formulating pesticides, herbicides, or fungicides -- i.e., horticulturally acceptable carriers -- are suitable.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements such as, for example, carbon and sulphur; natural and synthetic resins such as, for example, coumarone resins, polyvinyl chloride, and styrene polymers and copolymers; bitumen; waxes such as, for example, beeswax, paraffin wax, and chlorinated mineral waxes; solid fertilizers, for example, superphosphates; and ground, naturally-occurring, fibrous materials, such as ground corncobs.

Example of suitable liquid carriers are water, alcohols such as isopropyl alcohol and glycols; ketones such as acetone, methyl ethyl ketone, methyl isobutyl ketone and cyclohexanone; ethers such as cellosolves; aromatic hydrocarbons such as benzene, toluene and xylene; petroleum fractions such as kerosene and light mineral oils; chlorinated hydrocarbons such as carbon tetrachloride, perchloroethylene and trichloromethane. Also suitable are liquefied, normally vaporous and gaseous compounds. Mixtures of different liquids are often suitable.

The surface-active agent may be an emulsifying agent or a dispersing agent or a wetting agent; it may be nonionic or ionic. Any of the surface-active agents usually applied in formulating herbicides or insecticides may be used. Examples of suitable surface-active agents are the sodium and calcium salts of polyacrylic acids and lignin sulfonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example, p-octylphenol or p-octylcresol, with ethylene oxide and/or propylene oxide; sulfates or sulfonates of these condensation products, alkali or alkaline earth metal salts, preferably sodium salts, of sulfuric or sulfonic acid esters containing at least 10 carbon atoms in the molecule, for example, sodium lauryl sulfate, sodium secondary alkyl sulfates, sodium salts of sulfonated castor oil, and sodium alkylaryl sulfonates such as sodium dodecylbenzene sulfonate; and polymers of ethylene oxide and copolymers of ethylene oxide and propylene oxides.

The compositions of the invention may be prepared as wettable powders, dusts, granules, solutions, emulsifiable concentrates, emulsions, suspension concentrates and aerosols. Wettable powders are usually compounded to contain 25 to 75% by weight of active compound and usually contain, in addition to the solid carrier, 3-10% by weight of a dispersing agent, 2-15% of a surface-active agent and, where necessary, 0-10% by weight of stabilizer(s) and/or other additives such as penetrants or stickers. Dusts are usually formulated as a dust concentrate having a similar composition to that of a wettable powder but without a dispersant or surface-active, and are diluted in the field with further solid carrier to give a composition usually containing 0.5-10% by weight of the active compound. Granules are usually prepared to have a size between 10 and 100 BS mesh (1.676-0.152 mm), and may be manufactured by agglomeration or impregnation techniques. Generally, granules will contain 0.5-25% by weight of the active compound, 0-1% by weight of additives such as stabilizers, slow release modifiers and binding agents. Emulsifiable concentrates usually contain, in addition to the solvent and, when necessary, cosolvent, 10-50% weight per volume of the active compound, 2-20% weight per volume emulsifiers and 0-20% weight per volume of appropriate additives such as stabilizers, penetrants and corrosion inhibitors. Suspension concentrates are compounded so as to obtain a stable, non-sedimenting, flowable product and usually contain 10-75% weight of the active compound, 0.5-5% weight of dispersing agents, 1-5% of surface-active agent, 0.1-10% weight of suspending agents, such as defoamers, corrosion inhibitors, stabilizers, penetrants and stickers, and as carrier, water or an organic liquid in which the active compound is substantially insoluble; certain organic solids or inorganic salts may be dissolved in the carrier to assist in preventing sedimentation or as antifreeze agents for water.

Of particular interest in current practice are water-dispersible granular formulations. There are in the form of dry, hard granules that are essentially dust-free, and are resistant to attrition on handling, thus minimizing the formation of dust. On contact with water, the granules readily disintegrate to form stable suspensions of the particles of active material. Such formulations contain 90% or (up to 95%) more by weight of finely divided active material, 3-7% by weight of a blend of surfactants, which act as wetting, dispersing, suspending and binding agents, and may contain up to 3% by weight of a finely divided carrier, which acts as a resuspending agent.

Aqueous dispersions and emulsions, for example, compositions obtained by diluting a wettable powder

or a concentrate according to the invention with water, also lie within the scope of the present invention. The said emulsions may be of the water-in-oil or of the oil-in-water type, and may have thick, mayonnaise-like consistency.

It is evident from the foregoing that this invention contemplates compositions containing as little as about 0.5% by weight to as much as about 95% by weight of a compound of Formula I as the active ingredient.

The compositions of the invention may also contain other ingredients, for example, other compounds possessing pesticidal, especially insecticidal, acaricidal, herbicidal or fungicidal properties, as are appropriate to the intended purpose.

Protection of a locus or area from undesirable plants is effected by applying a compound of Formula I, ordinarily in a composition of one of the aforementioned types, to soil in which the seeds of the unwanted plants are present, or to the foliage of the unwanted plants. The active compound, of course, is applied in an amount sufficient to exert the desired action.

The amount of the compound of the invention to be used in combatting undesired plants will naturally depend on the condition of the plants, the degree of activity desired, the formulation used, the mode of application, the climate, the season of the year, and other variables. Recommendations as to precise amounts are, therefore, not possible. In general, however, application to the locus to be protected of from 0.02 to 10.0 kg per hectare of the compound of Formula I will be satisfactory.

The following examples describe the preparation, isolation and physical properties of typical individual examples of the compounds of Formula I. In each case, the identity of each product, and each of any intermediate involved was confirmed by elemental, infrared and nuclear magnetic resonance spectral (NMR) analysis as necessary.

Example 1 - N-(1-oxo-6-indanyl)-3,4,5,6-tetrahydrophthalimide (1)

26.4 g of 1-indanone was added in small portions over 30 minutes to 175 ml of vigorously stirred fuming nitric acid at -1 ° C to -9 ° C. The mixture was stirred for 10 more minutes in an ice bath, then poured onto 400 g of ice. The mixture was extracted with methylene chloride, and the extract was washed with 5% w:v aqueous sodium hydroxide solution. The organic phase was washed with water, then brine, dried ($Na_2SO_4$) and stripped of solvent. Flash chromatography on silica gel with ethyl acetate and hexane as eluent gave 1-oxo-6-nitroindane (1A), m.p.: 72-73.5 ° C.

12.5 g of 1A was dissolved in 150 ml of hot ethanol, the mixture was stirred vigorously while 2.0 g of 5% palladium-on-carbon catalyst was added, then 450 ml of a 30% solution of sodium hypophosphite in water was added in portions. The mixture was refluxed for 30 minutes, cooled to room temperature, and filtered through celite. The celite was washed with water and methylene chloride. The combined organic phases were separated, dried ($Na_2SO_4$) and stripped of solvent to give 6-amino-1-oxoindane (1B), as a green solid, m.p.: 147-155 ° C (with decomposition).

A mixture of 4.2 g of 1B and 4.34 g of 3,4,5,6-tetrahydrophthalic anhydride (THPA) in 40 ml of glacial acetic acid was heated at reflux. After 2 hours, the acetic acid was evaporated, and the residue was azeotroped with toluene to give a greenish solid. The solid was a triturated with ether, filtered, and recrystallized from ethyl acetate to give (1), as beige crystals; m.p.: 223-226 ° C.

Example 2 - N-(1-(allyloxyimino)-6-indanyl)-3,4,5,6-tetrahydrophthalimide (2)

A mixture of 0.28 g of (1), 0.11 g of allyloxyamine hydrochloride and 1.2 equivalents of triethylamine in ethanol was heated at reflux for 5 hours. The solvent was removed and the residue was chromatographed with a 3:7 v:v mixture of ethyl acetate and hexane as eluent to give 2, as the anti-isomer (2A), a white powder; m.p.: 133-135 ° C, and as the syn-isomer (2B), a yellow solid, m.p.: 117-119 ° C.

Example 3 - N-(1-(((ethoxycarbonyl)methoxy)imino)-6-indanyl)-3,4,5,6-tetrahydrophthalimide (3)

A stirred solution of 0.68 g of ((ethoxycarbonyl)methoxy)amine hydrochloride and 0.0044 M sodium acetate in 60 ml of absolute ethanol was warmed under nitrogen and 1.13 g of 1was added. The mixture was heated at reflux for 12 hours, then filtered and washed with ethanol. The total filtrate was evaporated and the residue was chromatographed with a 3:7 v:v mixture of ethyl acetate and hexane as eluent to give 3, as the anti-isomer (3A), a yellow solid, m.p.: 119-123 ° C, and as the syn-isomer: (3B), a white solid, m.p.: 121-125 ° C.

Example 4 - N-(1-(neopentoxyimino)-6-indanyl)-3,4,-5,6-tetrahydrophthalimide (4)

A solution of 1.3 g of 1and 0.78 g of neopentoxyamine oxalate in 20 ml of dry pyridine was stirred overnight at room temperature. The pyridine was evaporated and the residue was azeotroped thrice with toluene and flash chromatographed on silica gel with a 1:3 v:v mixture of ethyl acetate and hexane as eluent to give 4, as the anti-isomer (4A), a white solid; m.p.: 139-141 ° C, and as the syn-isomer (4B), a yellow solid; m.p.: 140-145 ° C.

Example 5 - N-(1-(methoxyimino)-6-indanyl)-3,4,5,6-tetrahydrophthalimide (5)

A mixture of 0.98 g of 1, 0.29 g of methoxyamine hydrochloride and 0.29 g of sodium acetate in 35 ml of ethyl alcohol was heated at reflux for six hours. The resulting mixture was flash chromatographed on silica gel with a 4:1 v:v mixture of hexane and ethyl acetate as eluent to give 5, as a yellow solid isomer mixture; m.p.: 168-178 ° C.

Example 6 - N-((1-(hydroxycarbonylmethoxy)imino)-6-indanyl)-3,4,5,6-tetrahydrophthalimide (6)

To a stirred solution of 0.56 g of aminoxyacetic acid hydrochloride in 20 ml of dry pyridine was added 1.13 g of 1. The mixture was stirred overnight, the pyridine was evaporated, and the residue was azeotroped thrice with toluene and vacuum pumped to about 0.1 Torr. at 60 ° C bath temperature. The resulting reddish oil was triturated with ethanol and the resulting light brown precipitate was filtered and dried to give 6, as the anti-isomer, a beige solid, m.p.: 205-206.5 ° C.

Example 7 - N-((1-((ethoxycarbonyl)-1-(1-ethoxycarbonyl)methoxy)imino)-6-indanyl)-3,4, -5,6-tetrahydrophthalimide (7)

A mixture of 0.120 g of 6, 0.05 ml of ethyl 2-bromopropionate and 52 mg of dry potassium carbonate in 2 ml of dry dimethylformamide was stirred at room temperature overnight. The mixture was poured into water and extracted thrice with ethyl acetate. The combined organic phase was washed with water and then brine, dried ($MgSO_4$), filtered and evaporated to give a yellow oil, which was chromatographed using a 1:9 v:v mixture of ethyl acetate and hexane as eluent to give 7, as the anti-isomer, a yellow oil.

Examples 8 and 9

Following procedures similar to those described in Examples 1-7, additional individual species of the invention were prepared from 1:

| Example No. | Compound | V | Y | a | m.p. (°C) |
|---|---|---|---|---|---|
| 8 | 8A | $=N-O-CH_2C\equiv CH$ (syn) | H | 1 | 169-170.5 |
| 8 | 8B | $=N-O-CH_2C\equiv CH$ (anti) | H | 1 | 154-156 |
| 9 | 9A | $=N-O-CH_2$phenyl (anti) | H | 1 | 147-148 |
| 9 | 9B | $=N-O-CH_2$phenyl (syn) | H | 1 | 131-133 |

Example 10 - N-(5-fluoro-1-oxo-6-indanyl)-3,4,5,6-tetrahydrophthalimide (10)

25.0 g of 5-fluoro-1-indanone was added dropwise to 115 ml of fuming nitric acid at 5±2 ° C, and the mixture was held at that temperature (ice bath) for 2 hours. The mixture was poured into 500 ml of ice-water and extracted with methylene chloride. The extract was washed with water, then brine, then dried ($Na_2SO_4$) and stripped of solvent. The residue was recrystallized from a minimum amount of ethanol, and the product was flash chromatographed on silica gel with a 35:65 v:v mixture of ethyl acetate and hexane as eluent to give 5-fluoro-6-nitro-1-indanone (10A), as beige crystals, m.p.: 63-65 ° C.

1.0 g of 10A was dissolved in 30 ml of ethyl acetate. 5.8 g of tin (II) chloride dihydrate was added and

the mixture was refluxed for 1 hour. The mixture was cooled to room temperature and poured into ice water. The pH of the mixture was brought to 12 with aqueous sodium hydroxide solution and extracted with ether. The extract was washed with water, then brine, then dried ($Na_2SO_4$) and stripped of solvent, to give 6-amino-5-fluoro-1-indanone (11B), as a tan solid, m.p.: 163-168°C (with decomposition).

A mixture of 3.41 g of THPA and 3.70 g of 10B in 50 ml of glacial acetic acid was refluxed for 6 hours and then allowed to sit for 48 hours. The mixture was poured into water and extracted thrice with ethyl acetate. The combined organic phase was washed with water, then brine, dried ($Na_2SO_4$), filtered, stripped of solvent and flash chromatographed using a 4:1 v:v mixture of ethyl acetate and hexane as eluent to give 10, as a tan solid, m.p.: 213-215°C.

Example 11 - N-(5-fluoro-1-(allyloxy)imino-6-indanyl)-3,4,5,6-tetrahydrophthalimide (11)

A mixture of 0.70 g of 10 and 0.260 g of allyloxyamine hydrochloride in 5 ml of dry pyridine was stirred overnight. The pyridine was evaporated and the residue flash chromatographed on silica with a 15:85 v:v mixture of ethyl acetate and hexane as eluent to give 11 as the anti-isomer (11A), white crystals, m.p.: 141-142.5°C and as the syn-isomer (11B), off-white solid, m.p.: 145-147°C.

Example 12 - N-((5-fluoro-1-((ethoxycarbonyl)-methoxy)-imino)-6-indanyl)-3,4,5,6-tetrahydrophthalimide (12)

A mixture of 1.0 g of 10, 0.52 g of ethyl aminoxyacetate hydrochloride and 10 ml of dry pyridine was stirred until the reaction was complete. The residue was flash chromatographed on silica gel 60 with a 3:7 v:v mixture of ethyl acetate and hexane as eluent to give 12, as the anti-isomer, a tan solid, m.p.: 160-161.5°C.

Example 13 - N-(1,2,3,4-tetrahydro-1-oxo-7-naphthalenyl)-3,4,5,6-tetrahydrophthalimide (13)

29.2 g of alpha-tetralone was slowly added to 200 ml of fuming nitric acid, cooled by an ice bath, at a rate such that the reaction mixture temperature did not exceed 8°C. Then the mixture was stirred in an ice bath for 30 minutes and poured onto 1.2 kg of ice. The mixture was filtered, and the collected solid was washed with water and dried. The solid was recrystallized from ethanol, then flash chromatographed on silica gel, being applied with methylene chloride and eluted with a 1:3 v:v mixture ethyl acetate and hexane, to give a fraction identified as 7-nitro-alpha-tetralone (13B), a beige solid, m.p.: 101-102.5°C.

13A was converted to 7-amino-alpha-tetralone (13B), as beige crystals, m.p.: 130-132°C, by the procedure described for converting 1A to 1B.

13 was obtained, as beige crystals, m.p.: 215-217°C, by treating THPA with 13B, according to the procedure described for converting 1B to 1.

Example 14 - N-((1-((ethoxycarbonyl)methoxy)imino)-1,2,3,4-tetrahydro-7-napthalenyl)- 3,4,5,6-tetrahydrophthalimide (14)

A mixture of 0.89 g of 13 and 0.51 g of (ethylaminoxyacetate hydrochloride in pyridine was stirred overnight. The pyridine was removed, the residue re-evaporated twice with toluene, and flash chromatographed on silica using 25% ethyl acetate in hexane as eluent to give 14, as the anti-isomer (14A), off-white cyrstals; m.p.: 99-101°C and an isomer mixture, which was rechromatographed twice using 15% ethyl acetate in hexane as eluent to give a 2:1 syn:anti-isomer mixture (14B), as a clear oil.

Examples 15 and 16

By procedures similar to those described in Examples 13 and 14, the following additional individual species were prepared from 13:

| Example No. | Compound | V | R | a | m.p. (°C) |
|---|---|---|---|---|---|
| 15 | 15A | $=N-O-CH_2CH=CH_2$ (syn) | H | 2 | 105-107 |
| 15 | 15B | $=N-OCH_2CH=CH_2$ (anti) | H | 2 | 108-110 |
| 16 | 16 | =N-OH (syn/anti) | H | 2 | 204-206 (with decomposition) |

Example 17 - N-((1-(2-tetrahydropyranyl)oxy)imino)-1,2,3,4-tetrahydro-7-naphthalenyl)-3,4,5,6-tetrahydrophthalimide (17)

17 was prepared as a yellow solid, melting point not determined, by treating 16 with 3,4-dihydro-2H-pyran in the presence of a small amount of p-toluenesulfonic acid.

Example 18 - N-(1-(hydroxyimino)-6-indanyl)-3,4,5,6-tetrahydrophthalimide (18)

A mixture of 1.03 g of 1B, 0.58g of hydroxylamine hydrochloride, 1.15g of sodium acetate and 50 ml of ethanol was refluxed for 3 hours, then stripped of volatiles. The residue was treated with water and methylene chloride, then filtered and dried in a vacuum oven to give 6-amino-1-(hydroxyimino)indane (18A), as a beige solid, m.p.: 151-153 °C.

A mixture of 0.46 g of THPA, 0.49 g of 18A and 10 ml of glacial acetic acid was refluxed for 2 hours. Then the solvent was stripped, and the residue was triturated with ether. The solid residue was air dried and identified as 18, a beige solid; melting point not determined.

By applying the procedures of Examples 1 to 18, the compounds in Tables 1 to 5 can be prepared by one skilled in the art.

General Formulae for Tables

General Formula 1

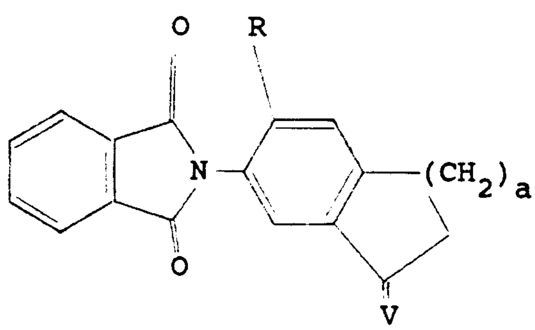

General Formula 2

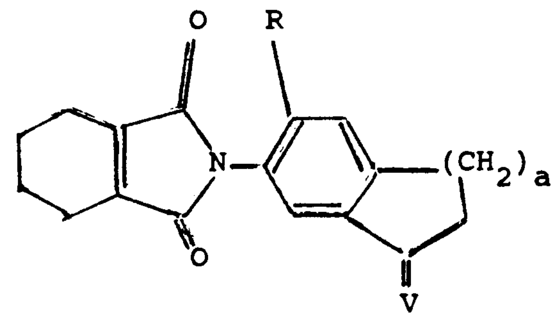

General Formula 3

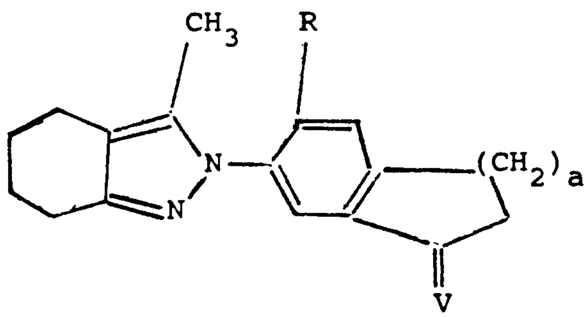

General Formula 4

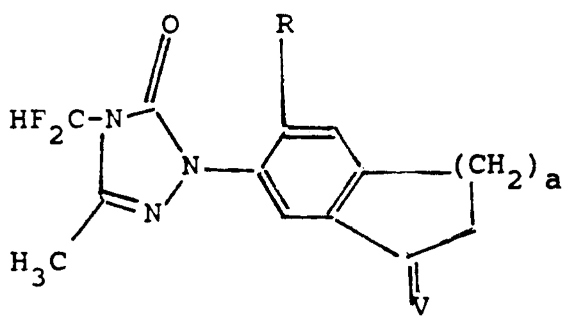

General Formula 5

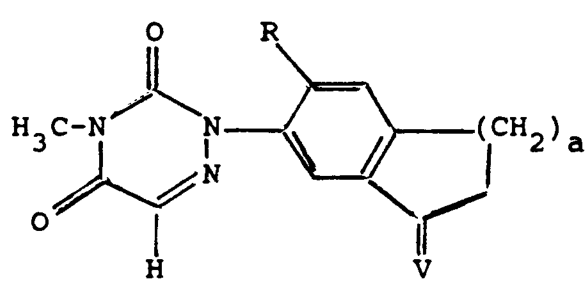

Table 1

General Formula 1

| R | a | V |
|---|---|---|
| H | 1 | $N-OCH_2CH_2CN$ |
| H | 1 | $N-OCH_2CH_2Cl$ |
| H | 1 | $N-OCH_2CH=CH_2$ |
| H | 1 | $N-OCH_2(CH_3)C=CH_2$ |
| H | 1 | $N-OCH_2(Cl)C=CH_2$ |
| H | 1 | $N-OCH_2CO_2H$ |
| H | 1 | $N-OCH_2CO_2C_2H_5$ |
| H | 1 | $N-OCH_2CO_2CH(CH_3)CO_2C_2H_5$ |
| H | 1 | $N-OCH_2(CH_3)CO_2CH_3$ |
| H | 1 | $N-OCH_2CH_2CH_2CH_3$ |
| H | 1 | $N-OCH(CH_3)CH_2CH_3$ |
| H | 1 | $N-OCH_2C(CH_3)_3$ |
| H | 1 | $N-OCH(CH_3)_2$ |
| H | 1 | $N-OCH_2-C_6H_5$ |
| H | 1 | $N-OCH_2-C_6H_4(4-Cl)$ |
| H | 1 | $N-OCH_2-C_6H_4(4-OCH_3)$ |
| H | 1 | O |
| H | 2 | $N-OCH_2CH_2CN$ |
| H | 2 | $N-OCH_2CH_2Cl$ |
| H | 2 | $N-OCH_2CH=CH_2$ |
| H | 2 | $N-OCH_2(CH_3)C=CH_2$ |
| H | 2 | $N-OCH_2(Cl)C=CH_2$ |
| H | 2 | $N-OCH_2CO_2H$ |
| H | 2 | $N-OCH_2CO_2C_2H_5$ |
| H | 2 | $N-OCH_2CO_2CH(CH_3)CO_2C_2H_5$ |
| H | 2 | $N-OCH(CH_3)CO_2CH_3$ |
| H | 2 | $N-OCH_2CH_2CH_2CH_3$ |
| H | 2 | $N-OCH(CH_3)CH_2CH_3$ |
| H | 2 | $N-OCH_2C(CH_3)_3$ |
| H | 2 | $N-OCH(CH_3)_2$ |
| H | 2 | $N-OCH_2-C_6H_5$ |
| H | 2 | $N-OCH_2-C_6H_4(4-Cl)$ |

| | | |
|---|---|---|
| H | 2 | $N-OCH_2-C_6H_4(4-OCH_3)$ |
| H | 2 | O |
| F | 2 | $N-OCH_2CH_2CN$ |
| F | 2 | $N-OCH_2CH_2Cl$ |
| F | 2 | $N-OCH_2CH=CH_2$ |
| F | 2 | $N-OCH_2(CH_3)C=CH_2$ |
| F | 2 | $N-OCH_2(Cl)C=CH_2$ |
| F | 2 | $N-OCH_2CO_2H$ |
| F | 2 | $N-OCH_2CO_2C_2H_5$ |
| F | 2 | $N-OCH_2CO_2CH(CH_3)CO_2C_2H_5$ |
| F | 2 | $N-OCH(OCH_3)CO_2CH_3$ |
| F | 2 | $N-OCH_2CH_2CH_2CH_3$ |
| F | 2 | $N-OCH(CH_3)CH_2CH_3$ |
| F | 2 | $N-OCH_2C(CH_3)_3$ |
| F | 2 | $N-OCH(CH_3)_2$ |
| F | 2 | $N-OCH_2-C_6H_5$ |
| F | 2 | $N-OCH_2-C_6H_4(4-Cl)$ |
| F | 2 | $N-OCH_2-C_6H_4(4-OCH_3)$ |
| F | 2 | O |

Table 2

General Formula 2

| R | a | V |
|---|---|---|
| H | 1 | N-$OCH_2CH_2CN$ |
| H | 1 | N-$OCH_2CH_2Cl$ |
| H | 1 | N-$OCH_2CH=CH_2$ |
| H | 1 | N-$OCH_2(CH_3)C=CH_2$ |
| H | 1 | N-$OCH_2(Cl)C=CH_2$ |
| H | 1 | N-$OCH_2CO_2H$ |
| H | 1 | N-$OCH_2CO_2C_2H_5$ |
| H | 1 | N-$OCH_2CO_2CH(CH_3)CO_2C_2H_5$ |
| H | 1 | N-$OCH(CH_3)CO_2CH_3$ |
| H | 1 | N-$OCH_2CH_2CH_2CH_3$ |
| H | 1 | N-$OCH(CH_3)CH_2CH_3$ |
| H | 1 | N-$OCH_2C(CH_3)_3$ |
| H | 1 | N-$OCH(CH_3)_2$ |
| H | 1 | N-$OCH_2$-$C_6H_5$ |
| H | 1 | N-$OCH_2$-$C_6H_4$(4-Cl) |
| H | 1 | N-$OCH_2$-$C_6H_4$(4-$OCH_3$) |
| H | 1 | O |
| F | 1 | N-$OCH_2CH_2CN$ |
| F | 1 | N-$OCH_2CH_2Cl$ |
| F | 1 | N-$OCH_2CH=CH_2$ |
| F | 1 | N-$OCH_2(CH_3)C=CH_2$ |
| F | 1 | N-$OCH_2(Cl)C=CH_2$ |
| F | 1 | N-$OCH_2CO_2H$ |
| F | 1 | N-$OCH_2CO_2C_2H_5$ |
| F | 1 | N-$OCH_2CO_2CH(CH_3)CO_2C_2H_5$ |
| F | 1 | N-$OCH(CH_3)CO_2CH_3$ |
| F | 1 | N-$OCH_2CH_2CH_2CH_3$ |
| F | 1 | N-$OCH(CH_3)CH_2CH_3$ |
| F | 1 | N-$OCH_2C(CH_3)_3$ |
| F | 1 | N-$OCH(CH_3)_2$ |
| F | 1 | N-$OCH_2$-$C_6H_5$ |
| F | 1 | N-$OCH_2$-$C_6H_4$(4-Cl) |

| | | |
|---|---|---|
| F | 1 | $N-OCH_2-C_6H_4(4-OCH_3)$ |
| F | 1 | O |
| H | 2 | $N-OCH_2CH_2CN$ |
| H | 2 | $N-OCH_2CH_2Cl$ |
| H | 2 | $N-OCH_2CH=CH_2$ |
| H | 2 | $N-OCH_2(CH_3)C=CH_2$ |
| H | 2 | $N-OCH_2(Cl)C=CH_2$ |
| H | 2 | $N-OCH_2CO_2H$ |
| H | 2 | $N-OCH_2CO_2C_2H_5$ |
| H | 2 | $N-OCH_2CO_2CH(CH_3)CO_2C_2H_5$ |
| H | 2 | $N-OCH(CH_3)CO_2CH_3$ |
| H | 2 | $N-OCH_2CH_2CH_2CH_3$ |
| H | 2 | $N-OCH(CH_3)CH_2CH_3$ |
| H | 2 | $N-OCH_2C(CH_3)_3$ |
| H | 2 | $N-OCH(CH_3)_2$ |
| H | 2 | $N-OCH_2-C_6H_5$ |
| H | 2 | $N-OCH_2-C_6H_4(4-Cl)$ |
| H | 2 | $N-OCH_2-C_6H_4(4-OCH_3)$ |
| H | 2 | O |
| F | 2 | $N-OCH_2CH_2CN$ |
| F | 2 | $N-OCH_2CH_2Cl$ |
| F | 2 | $N-OCH_2CH=CH_2$ |
| F | 2 | $N-OCH_2(CH_3)C=CH_2$ |
| F | 2 | $N-OCH_2(Cl)C=CH_2$ |
| F | 2 | $N-OCH_2CO_2H$ |
| F | 2 | $N-OCH_2CO_2C_2H_5$ |
| F | 2 | $N-OCH_2CO_2CH(CH_3)CO_2C_2H_5$ |
| F | 2 | $N-OCH(CH_3)CO_2CH_3$ |
| F | 2 | $N-OCH_2CH_2CH_2CH_3$ |
| F | 2 | $N-OCH(CH_3)CH_2CH_3$ |
| F | 2 | $N-OCH_2C(CH_3)_3$ |
| F | 2 | $N-OCH(CH_3)_2$ |
| F | 2 | $N-OCH_2-C_6H_5$ |
| F | 2 | $N-OCH_2-C_6H_4(4-Cl)$ |
| F | 2 | $N-OCH_2-C_6H_4(4-OCH_3)$ |
| F | 2 | O |

Table 3

General Formula 3

| R | a | V |
|---|---|---|
| H | 1 | $N-OCH_2CH_2CN$ |
| H | 1 | $N-OCH_2CH_2Cl$ |
| H | 1 | $N-OCH_2CH=CH_2$ |
| H | 1 | $N-OCH_2(CH_3)C=CH_2$ |
| H | 1 | $N-OCH_2(Cl)C=CH_2$ |
| H | 1 | $N-OCH_2CO_2H$ |
| H | 1 | $N-OCH_2CO_2C_2H_5$ |
| H | 1 | $N-OCH_2CO_2CH(CH_3)CO_2C_2H_5$ |
| H | 1 | $N-OCH(CH_3)CO_2CH_3$ |
| H | 1 | $N-OCH_2CH_2CH_2CH_3$ |
| H | 1 | $N-OCH(CH_3)CH_2CH_3$ |
| H | 1 | $N-OCH_2C(CH_3)_3$ |
| H | 1 | $N-OCH(CH_3)_2$ |
| H | 1 | $N-OCH_2-C_6H_5$ |
| H | 1 | $N-OCH_2-C_6H_4(4-Cl)$ |
| H | 1 | $N-OCH_2-C_6H_4(4-OCH_3)$ |
| H | 1 | O |
| H | 2 | $N-OCH_2CH=CH_2$ |
| H | 2 | $N-OCH_2(CH_3)C=CH_2$ |
| H | 2 | $N-OCH_2(Cl)C=CH_2$ |
| H | 2 | $N-OCH_2CO_2H$ |
| H | 2 | $N-OCH_2CO_2C_2H_5$ |
| H | 2 | $N-OCH(CH_3)CO_2CH_3$ |
| H | 2 | $N-OCH(CH_3)_2$ |
| H | 2 | $N-OCH_2C_6H_5$ |
| F | 1 | $N-OCH_2CH=CH_2$ |
| F | 1 | $N-OCH_2(CH_3)C=CH_2$ |
| F | 1 | $N-OCH_2(Cl)C=CH_2$ |
| F | 1 | $N-OCH_2CO_2H$ |
| F | 1 | $N-OCH_2CO_2C_2H_5$ |
| F | 1 | $N-OCH(CH_3)CO_2CH_3$ |
| F | 1 | $N-OCH(CH_3)_2$ |

| | | |
|---|---|---|
| F | 1 | $N-OCH_2C_6H_5$ |
| H | 2 | $N-OCH_2CH=CH_2$ |
| H | 2 | $N-OCH_2(Cl)C=CH_2$ |
| H | 1 | $N-OCH_2CO_2C_2H_5$ |
| H | 1 | $N-OCH(CH_3)_2$ |
| F | 2 | $N-OCH_2CH=CH_2$ |
| F | 2 | $N-OCH_2(Cl)C=CH_2$ |
| F | 2 | $N-OCH_2CO_2C_2H_5$ |
| F | 2 | $N-OCH(CH_3)_2$ |
| H | 1 | O |
| H | 2 | O |
| F | 1 | O |
| F | 2 | O |

Table 4

General Formula 4

| R | a | V |
|---|---|---|
| H | 1 | $N-OCH_2CH=CH_2$ |
| H | 1 | $N-OCH_2(CH_3)C=CH_2$ |
| H | 1 | $N-OCH_2(Cl)C=CH_2$ |
| H | 1 | $N-OCH_2CO_2H$ |
| H | 1 | $N-OCH_2CO_2C_2H_5$ |
| H | 1 | $N-OCH(CH_3)CO_2CH_3$ |
| H | 1 | $N-OCH(CH_3)_2$ |
| H | 1 | $N-OCH_2C_6H_5$ |
| F | 1 | $N-OCH_2CH=CH_2$ |
| F | 1 | $N-OCH_2(CH_3)C=CH_2$ |
| F | 1 | $N-OCH_2(Cl)C=CH_2$ |
| F | 1 | $N-OCH_2CO_2H$ |
| F | 1 | $N-OCH_2CO_2C_2H_5$ |
| F | 1 | $N-OCH(CH_3)CO_2CH_3$ |
| F | 1 | $N-OCH(CH_3)_2$ |
| F | 1 | $N-OCH_2C_6H_5$ |
| H | 2 | $N-OCH_2CH=CH_2$ |
| H | 2 | $N-OCH_2(Cl)C=CH_2$ |
| H | 2 | $N-OCH_2CO_2C_2H_5$ |
| H | 2 | $N-OCH(CH_3)_2$ |
| F | 2 | $N-OCH_2CH=CH_2$ |
| F | 2 | $N-OCH_2(Cl)C=CH_2$ |
| F | 2 | $N-OCH_2CO_2C_2H_5$ |
| F | 2 | $N-OCH(CH_3)_2$ |
| H | 1 | O |
| H | 2 | O |
| F | 1 | O |
| F | 2 | O |

Table 5

General Formula 5

| R | a | V |
|---|---|---|
| H | 1 | N-$OCH_2CH=CH_2$ |
| H | 1 | N-$OCH_2(CH_3)C=CH_2$ |
| H | 1 | N-$OCH_2(Cl)C=CH_2$ |
| H | 1 | N-$OCH_2CO_2H$ |
| H | 1 | N-$OCH_2CO_2C_2H_5$ |
| H | 1 | N-$OCH(CH_3)CO_2CH_3$ |
| H | 1 | N-$OCH(CH_3)_2$ |
| H | 1 | N-$OCH_2C_6H_5$ |
| F | 1 | N-$OCH_2CH=CH_2$ |
| F | 1 | N-$OCH_2(CH_3)C=CH_2$ |
| F | 1 | N-$OCH_2(Cl)C=CH_2$ |
| F | 1 | N-$OCH_2CO_2H$ |
| F | 1 | N-$OCH_2CO_2C_2H_5$ |
| F | 1 | N-$OCH(CH_3)CO_2CH_3$ |
| F | 1 | N-$OCH(CH_3)_2$ |
| F | 1 | N-$OCH_2C_6H_5$ |
| H | 2 | N-$OCH_2CH=CH_2$ |
| H | 2 | N-$OCH_2(Cl)C=CH_2$ |
| H | 2 | N-$OCH_2CO_2C_2H_5$ |
| H | 2 | N-$OCH(CH_3)_2$ |
| F | 2 | N-$OCH_2CH=CH_2$ |
| F | 2 | N-$OCH_2(Cl)C=CH_2$ |
| F | 2 | N-$OCH_2CO_2C_2H_5$ |
| F | 2 | N-$OCH(CH_3)_2$ |
| H | 1 | O |
| H | 2 | O |
| F | 1 | O |
| F | 2 | O |

Example 19 - Herbicidal Activity

In the following examples, the species of plants that were tested were:

| | Abbreviation |
|---|---|
| Barnyardgrass (water grass) - Echinochloa crus-galli | BYGR |
| Downy brome - Bromus tectorum | DOBR |
| Yellow foxtail - Setaria glauca | YEFT |
| Sicklepod - Cassia obtusifolia | SIPO |
| Velvetleaf - Abutilon theophrasti | VELE |
| Garden cress - Lepidium sativum | GACR |
| Johnsongrass - Sorghum halepense | JOGR |
| Morninglory - Ipomoea sp. | MOGL |
| Field bindweed - Convolvulus arvensis | FIBW |
| Nightshade - Solanum sp. | NISH |
| Blackgrass - Alopecurus myosuroides | BLGR |
| Yellow millet - Panicum miliceum | YEMI |
| Large crabgrass - Digitaria sanguinalis | LACG |
| Redroot pigweed - Amaranthus retroflexus | RRPW |
| Hemp sesbania - Sesbania exaltata | HESE |
| Prickly sida - Sida spinosa | PRSI |

Test Procedures

The pre-emergence (soil) herbicidal activity of compounds of Formula I was evaluated by planting seeds of downy brome, johnsongrass, yellow foxtail, barnyardgrass, yellow millet, blackgrass, hemp sesbania, velvetleaf, morninglory, prickly sida, sicklepod and garden cress in test tubes, nominally measuring 25× 200 millimeters, filled about three-quarters full of untreated soil, in each case covered on top with about 2.5 cubic centimetres of soil, treated with 0.1 milligram of the test compound, to give a dosage of 1.12 kg/ha of test compound (1.0 pound per acre). The seeds were planted on top of the treated soil and covered with about 1.5 cubic centimetres of untreated soil. The planted soil was held under a controlled regimen of temperature, moisture, and light. At 10 days, the amounts of germination and growth in each tube were evaluated on a 0 to 9 scale, the numeric ratings having the following meanings:

| Rating | Meaning |
|---|---|
| 9 | No living tissue |
| 8 | Plant severely damaged and expected to die |
| 7 | Plant badly damaged, but expected to live |
| 6 | Moderate damage, but complete recovery expected |
| 5 | Intermediate damage (probably unacceptable for crop plants) |
| 3-4 | Observable damage |
| 1-2 | Plant slightly affected, possibly by the chemical, possibly due to biological variability |
| 0 | No visible effect |

The postmergence (foliar) herbicidal activity of compounds of Formula I was evaluated by spraying 9-day-old large crabgrass plants, 9-day-old pigweed plants, 6-day-old johnsongrass plants, 9-day-old velvetleaf plants, 8-day-old yellow foxtail plants, 9-day-old sicklepod plants, 5-day-old morninglory plants, 5-day-old barnyardgrass plants, 6-day-old yellow millet plants, 9-day-old nightshade plants, 9-day-old prickly sida plants and 7-day-old field bindweed plants to runoff with 2.4 millilitres of a liquid formulation containing 0.5 milligram of the test compound (1.12 kg of the test compound per hectare). The sprayed plants were held under a controlled regimen of temperature, moisture and light for 7 to 8 days, when the effect of the test compound was evaluated visually, the results being rated on the 0 to 9 scale described above.

Results of the postemergence herbicidal activity tests are set forth in Tables I and II.

Table 1 - Postemergence Herbicidal Activity

| Compound No. | LACG | JOGR | YEFT | BYGR | YEMI | RRPW | NISH | VELE | MOGL | PRSI | SIPO | FIBW |
|---|---|---|---|---|---|---|---|---|---|---|---|---|
| 5 | - | 5 | 3 | -a) | - | - | - | 4 | 8 | - | - | - |
| 1 | - | 2 | 2 | - | - | - | - | 3 | 2 | - | - | - |
| 18 | - | 4 | 2 | - | - | - | - | 3 | 4 | - | - | - |
| 2A | - | 5 | 5 | - | - | - | - | 5 | 6 | - | - | - |
| 2B | - | 6 | 9 | - | - | - | - | 7 | 8 | - | - | - |
| 3A | 3 | 9 | 2 | 2 | 4 | 8 | 9 | 4 | 9 | 6 | 3 | 9 |
| 3B | 3 | 9 | 2 | 2 | 3 | 9 | 9 | 9 | 9 | 9 | 4 | 9 |
| 4A | 5 | 5 | 5 | 5 | 7 | 9 | 9 | 5 | 8 | 9 | 2 | 7 |
| 4B | 5 | 0 | 4 | 3 | 5 | 7 | 9 | 4 | 8 | 4 | 3 | 7 |
| 6 | 3 | 7 | 3 | 2 | 2 | 8 | 6 | 4 | 9 | 4 | 4 | 9 |
| 7 | 3 | 4 | 7 | 0 | 5 | 9 | 9 | 5 | 9 | 4 | 3 | 9 |
| 8A | 3 | 4 | 9 | 3 | 3 | 8 | 9 | 3 | 9 | 5 | 3 | 3 |
| 8B | 5 | 7 | 9 | 3 | 4 | 5 | 9 | 5 | 9 | 8 | 3 | 9 |
| 9A | 5 | 7 | 9 | 9 | 4 | 8 | 9 | 6 | 9 | 7 | 3 | 9 |
| 9B | 4 | 8 | 7 | 3 | 3 | 5 | 9 | 5 | 7 | 5 | 3 | 7 |
| 14A | 2 | 4 | 3 | 2 | 3 | 9 | 9 | 4 | 9 | 8 | 3 | 9 |

Table 1 - Postemergence Herbicidal Activity (cont'd)

| Compound No. | LACG | JOGR | YEFT | BYGR | YEMI | RRPW | NISH | VELE | MOGL | PRSI | SIPO | FIBW |
|---|---|---|---|---|---|---|---|---|---|---|---|---|
| 14B | 2 | 5 | 2 | 6 | 3 | 9 | - | 8 | 9 | 9 | 3 | 9 |
| 15A | 3 | 3 | 4 | 5 | 4 | 9 | 9 | 7 | 9 | 6 | 4 | 7 |
| 15B | 3 | 4 | 7 | 5 | 3 | 5 | 9 | 4 | 7 | 3 | 4 | 5 |
| 13 | 0 | 0 | 0 | 0 | 3 | 0 | 7 | 0 | 5 | 0 | 0 | 3 |
| 10 | 5 | 3 | 5 | 3 | 7 | 9 | 9 | 9 | 9 | 6 | 0 | 4 |
| 11A | 4 | 8 | 9 | 6 | 7 | 9 | 9 | 7 | 9 | 8 | 5 | 9 |
| 11B | 7 | 6 | 9 | 8 | 8 | 9 | 9 | 8 | 9 | 9 | 5 | 9 |
| 17 | 4 | 4 | 7 | 3 | 3 | 8 | 8 | 4 | 8 | 6 | 3 | 7 |
| 12 | 4 | 8 | 9 | 4 | - | 7 | - | 9 | 9 | - | 5 | - |

a) - indicates that the compound was not tested with respect to this species.

## Claims

**Claims for the following Contracting States : AT, BE, CH, DE, FR, GB, GR, IT, LI, LU, NL, SE**

**1.** A compound of the formula:

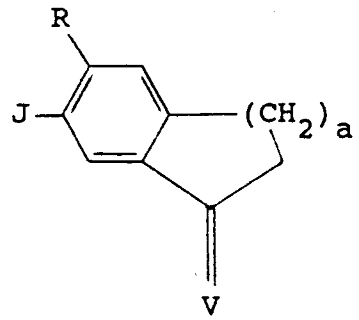

(I)

wherein

R is a hydrogen or halogen atom; a cyano group; an alkyl or alkoxy group having one to four carbon atoms; a trihalomethyl or hydroxymethyl group; an alkyl-, alkenyl-, or alkanoyl-oxymethyl group of up to six carbon atoms; or a group $-OCF_nH_{3-n}$ wherein n = one, two or three;

V is an oxygen atom or a group $N-O-R^1$, wherein $R^1$ represents a hydrogen atom; an alkyl, alkenyl or alkynyl group having up to six carbon atoms, the alkyl group optionally being substituted by cyano, mono- or poly- halo, or hydroxy- or alkoxy- carbonyl and the alkenyl group being optionally mono- or poly- halo-substituted; a phenyl group optionally substituted by one or more groups $R^{14}$ as defined below or a phenylalkyl group having seven to ten carbon atoms optionally substituted with one or more groups $R^{14}$ as defined below; or a heteroaryl or heteroaralkyl group wherein the heteroaryl moiety is furanyl, pyrrolyl, thienyl, pyridinyl, thiazolyl, isoxazolyl, pyrazolyl, imidazolyl, triazolyl (1,2,3- or 1,2,4-), benzofuranyl, indolyl, indazolyl, thianaphthenyl, pyridazinyl, pyrimidinyl, pyrazinyl, cinnolinyl, quinazolinyl or quinoxalinyl;

a is one or two;

J is one of the moieties:

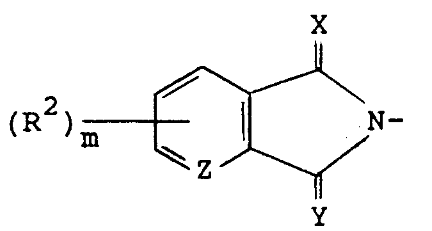

J-1

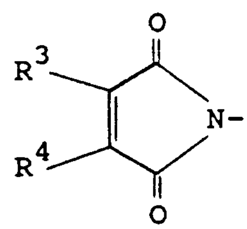

J-2

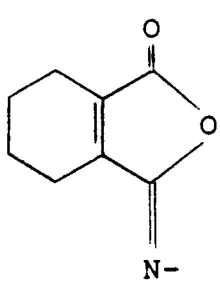

J-3

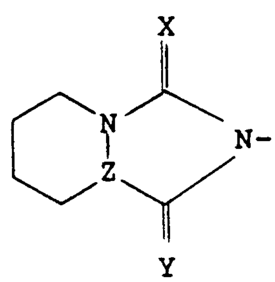

J-4

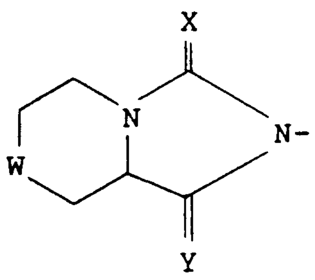

J-5

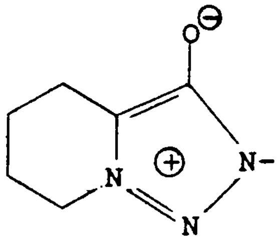

J-6

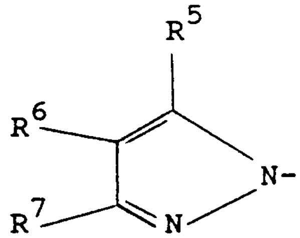

J-7

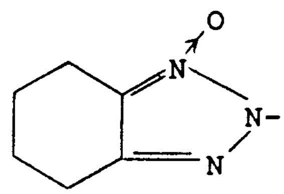

J-8

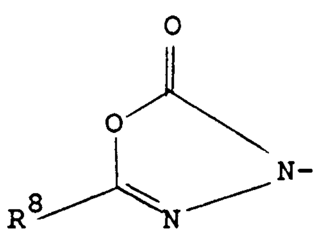

J-9

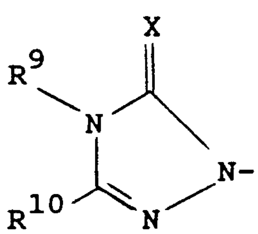

J-10

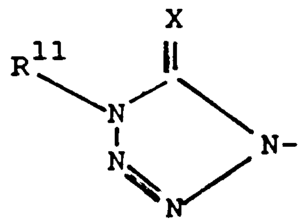

J-11

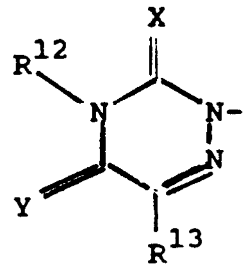

J-12

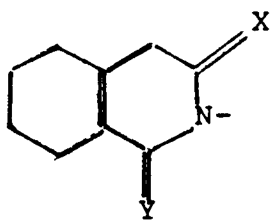

J-13

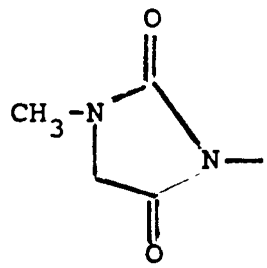

J-14

wherein

X and Y each independently represents O or S;

Z is CH or N;

W is S or $SO_2$;

m is zero, one or two;

$R^2$ is a halogen atom, or is an alkyl, haloalkyl or alkoxy group having one to four carbon atoms;

$R^3$ and $R^4$ each independently represents a hydrogen atom or an alkyl group having one to four carbon atoms, or $R^3$ and $R^4$ together with the carbon atoms to which they are attached form an alicyclic six-membered ring that may be substituted by one to three methyl group;

$R^5$ is a hydrogen or halogen atom, or an alkyl or alkyl-$S(O)_b$- group having one to four carbon atoms wherein b is zero, one or two;

$R^6$ is a hydrogen or halogen atom, or an alkyl, alkylthio or alkylsulfonyl group having one to four carbon atoms;

$R^7$ is an alkyl group having one to four carbon atoms, or $R^6$ and $R^7$ together form the moiety, $-(CH_2)_c-$, wherein c is three or four, which may be substituted by one to three methyl group;

$R^8$ is a hydrogen atom or an alkyl, alkenyl, haloalkyl or alkoxyalkyl group having up to six carbon atoms, or a cycloalkyl group having three to six carbon atoms, a cycloalkylalkyl group having four to eight carbon atoms, or either of these groups substituted by one to three alkyl groups each having one to four carbon atoms;

$R^9$ has one to six carbon atoms and is an alkyl, haloalkyl, cyanoalkyl, alkoxyalkyl, alkenyl, alkynyl, or -alkyl-$S(O)_a$-alkyl group where a is zero, one or two;

$R^{10}$ is a hydrogen atom, a group $R^9$ as defined above, or $R^9$ and $R^{10}$ together represent an alkylene moiety $-(CH_2)_d-$, as defined above for $R^7$, or a moiety $-(CH_2)_c-U-(CH_2)_e-$wherein d and e each is zero, one, two or three, and d plus e = two or three, and U is oxygen, $-S(O)_a-$or -NRalkyl where a is zero, one or two with the proviso that when U is $-S(O)_a-$, d is other than zero;

$R^{11}$ has one to six carbon atoms, and is an alkyl, haloalkyl, alkoxyalkyl, alkylthioalkyl, cyano- alkyl, haloalkoxyalkyl, alkenyl or haloalkenyl group;

$R^{12}$ is a hydrogen atom, or an amino group, or has one to four carbon atoms and is an alkyl, haloalkyl, cyanoalkyl, alkenyl, alkynyl, alkoxyalkyl, or alkoxycarbonyl group.

$R^{13}$ is a group $R^{11}$ as defined above, or is a hydrogen atom, or a carboxyl or alkoxycarbonyl group;

$R^{14}$ is a halogen atom, or a $C_1$ to $C_3$ alkyl, $C_1$ to $C_3$ alkoxy, $CF_3$, $NO_2$, CN or $OCF_2H$ group;

and salts of the acidic species with alkali metal bases, ammonia and amines.

**2.** Compounds of Claim 1 where
R is a hydrogen or halogen atom or a $C_1$ to $C_3$ alkyl group; $R^1$ is a hydrogen atom or a $C_1$ to $C_6$ alkyl, $C_3$ to $C_5$ alkenyl, $C_3$ to $C_5$ alkynyl, $C_3$ to $C_8$ alkoxycarbonylalkyl, or benzyl group; and $R^{14}$ is a fluorine or chlorine atom or a $CH_3$ or $OCH_3$ group.

**3.** Compounds of Claim 2, where a is one.

**4.** Compounds of Claim 2, where a is two.

**5.** Compounds of Claim 2, where $R^2$ is a fluorine or chlorine atom or a $CH_3$ or $OCH_3$ group; m is 0 or 1; R is a hydrogen, fluorine or chlorine atom or a $C_1$ to $C_3$ alkyl group; $R^1$ is a hydrogen atom or a $C_1$ to $C_4$ alkyl, allyl, propargyl, optionally substituted benzyl, or $C_3$ to $C_8$ alkoxycarbonylalkyl group.

**6.** Compounds of Claim 5, where
m is 0;
V is $=NOR^1$;
X and Y represent oxygen atoms; and
R is a hydrogen, fluorine or chlorine atom or a $CH_3$ group.

**7.** Compounds of Claim 6, where $R^3$ and $R^4$ form an alicyclic six-membered ring;
R is a hydrogen atom or a $CH_3$ group;
$R^1$ is a hydrogen atom or a $C_1$ to $C_4$ alkyl, allyl, propargyl, optionally substituted benzyl, or $C_3$ to $C_8$ alkoxycarbonylalkyl group.

**8.** Compounds of Claim 7, where R is a hydrogen or fluorine atom; and $R^3$ and $R^4$ form $-(CH_2)_4-$.

**9.** A compound of the formula

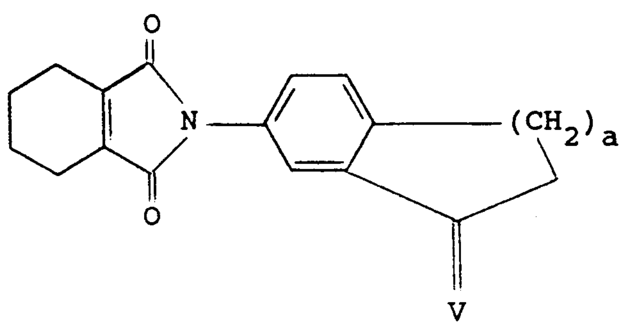

wherein V is -N-O-R", and either a is 1 and R" is an (ethoxycarbonyl)-1-(1-ethoxycarbonyl)methyl group or a is 2 and R" is a tetrahydropyranyl group.

**10.** A process for the preparation of a compound of formula I as defined in any one of the preceding claims comprising treating an aniline or isocyanate of formula II:

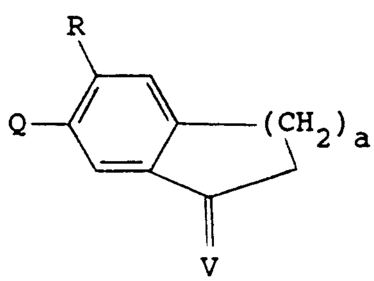

(II)

wherein Q is $-NH_2$ or -NCO with the appropriate reagents to introduce any one of the groups J-1 to J-14 and, optionally, oximating a compound of formula I where V is an oxygen atom to a compound of Formula I where V is the group $N-O-R^1$.

**11.** A compound as defined in any one of the preceding claims 1 to 9 when prepared by the process of claim 10.

**12.** A composition suitable for controlling the growth of undesired vegetation which comprises an effective amount of a compound as claimed in any one of claims 1 to 9 or 11 and at least one surfactant, solid or liquid diluent.

**13.** A method for controlling the growth of undesired vegetation which comprises applying to the locus to be protected an effective amount of a compound as claimed in any one of claims 1 to 9 or 11 or a composition as claimed in claim 12.

**14.** The use of a compound as claimed in any one of claims 1 to 9 or 11 as a herbicide.

**Claims for the following Contracting State : ES**

**1.** A process for the preparation of a compound of the formula:

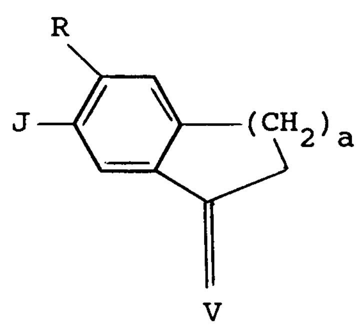

(I)

wherein

R is a hydrogen or halogen atom; a cyano group; an alkyl or alkoxy group having one to four carbon atoms; a trihalomethyl or hydroxymethyl group; an alkyl-, alkenyl-, or alkanoyl-oxymethyl group of up to six carbon atoms; or a group $-OCF_nH_{3-n}$ wherein n = one, two or three;

V is an oxygen atom or a group $N-O-R^1$, wherein $R^1$ represents a hydrogen atom; an alkyl, alkenyl or alkynyl group having up to six carbon atoms, the alkyl group optionally being substituted by cyano, mono- or poly- halo, or hydroxy- or alkoxy- carbonyl and the alkenyl group being optionally mono- or poly- halo-substituted; a phenyl group optionally substituted by one or more groups $R^{14}$ as defined below or a phenylalkyl group having seven to ten carbon atoms optionally substituted with one or more groups $R^{14}$ as defined below; or a heteroaryl or heteroaralkyl group wherein the heteroaryl moiety is furanyl, pyrrolyl, thienyl, pyridinyl, thiazolyl, isoxazolyl, pyrazolyl, imidazolyl, triazolyl (1,2,3- or 1,2,4-), benzofuranyl, indolyl, indazolyl, thianaphthenyl, pyridazinyl, pyrimidinyl, pyrazinyl, cinnolinyl, quinazolinyl or quinoxalinyl;

a is one or two;

J is one of the moieties:

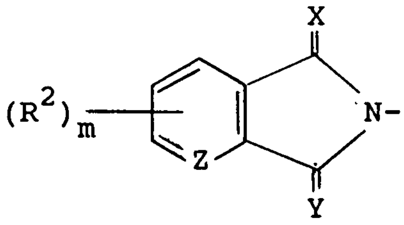

J-1

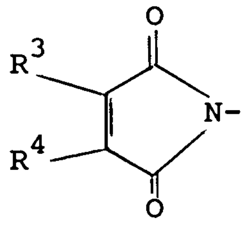

J-2

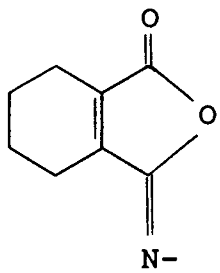

J-3

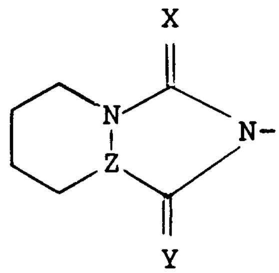

J-4

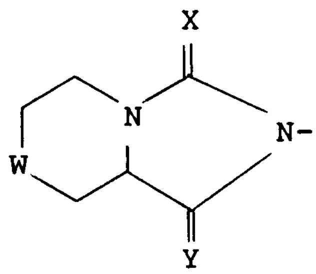

J-5

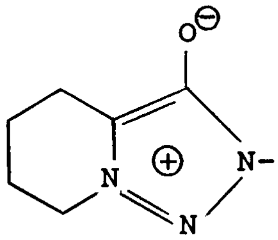

J-6

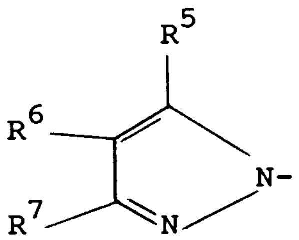

J-7

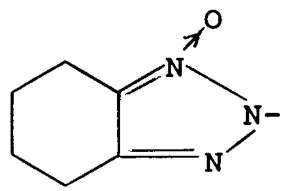

J-8

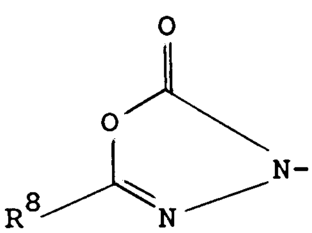

J-9

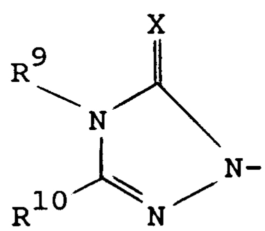

J-10

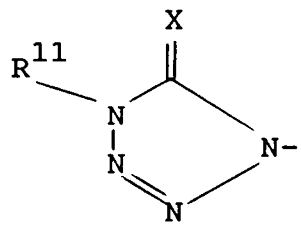

J-11

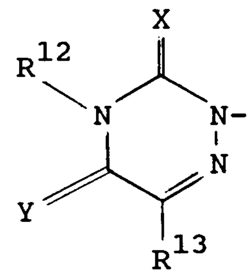

J-12

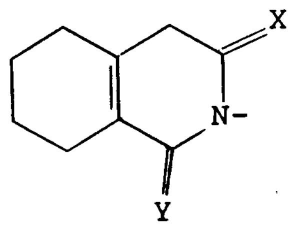

J-13

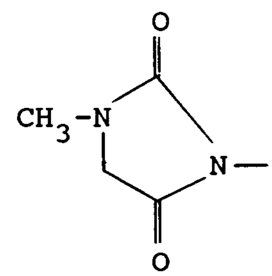

J-14

wherein
X and Y each independently represents O or S;
Z is CH or N;
W is S or $SO_2$;
m is zero, one or two;

$R^2$ is a halogen atom, or is an alkyl, haloalkyl or alkoxy group having one to four carbon atoms;

$R^3$ and $R^4$ each independently represents a hydrogen atom or an alkyl group having one to four carbon atoms, or $R^3$ and $R^4$ together with the carbon atoms to which they are attached form an alicyclic six-membered ring that may be substituted by one to three methyl group;

$R^5$ is a hydrogen or halogen atom, or an alkyl or alkyl-$S(O)_b$- group having one to four carbon atoms wherein b is zero, one or two;

$R^6$ is a hydrogen or halogen atom, or an alkyl, alkylthio or alkylsulfonyl group having one to four carbon atoms;

$R^7$ is an alkyl group having one to four carbon atoms, or $R^6$ and $R^7$ together form the moiety, $-(CH_2)_c-$, wherein c is three or four, which may be substituted by one to three methyl group;

$R^8$ is a hydrogen atom or an alkyl, alkenyl, haloalkyl or alkoxyalkyl group having up to six carbon atoms, or a cycloalkyl group having three to six carbon atoms, a cycloalkylalkyl group having four to eight carbon atoms, or either of these groups substituted by one to three alkyl groups each having one to four carbon atoms;

$R^9$ has one to six carbon atoms and is an alkyl, haloalkyl, cyanoalkyl, alkoxyalkyl, alkenyl, alkynyl, or -alkyl-$S(O)_a$-alkyl group where a is zero, one or two;

$R^{10}$ is a hydrogen atom, a group $R^9$ as defined above, or $R^9$ and $R^{10}$ together represent an alkylene moiety $-(CH_2)_c-$, as defined above for $R^7$, or a moiety $-(CH_2)_d-U-(CH_2)_e-$ wherein d and e each is zero, one, two or three, and d plus e = two or three, and U is oxygen, $-S(O)_a-$ or -NRalkyl where a is zero, one or two with the proviso that when U is $-S(O)_a-$, d is other than zero;

$R^{11}$ has one to six carbon atoms, and is an alkyl, haloalkyl, alkoxyalkyl, alkylthioalkyl, cyano- alkyl, haloalkoxyalkyl, alkenyl or haloalkenyl group;

$R^{12}$ is a hydrogen atom, or an amino group, or has one to four carbon atoms and is an alkyl, haloalkyl, cyanoalkyl, alkenyl, alkynyl, alkoxyalkyl, or alkoxycarbonyl group.

$R^{13}$ is a group $R^{11}$ as defined above, or is a hydrogen atom, or a carboxyl or alkoxycarbonyl group;

$R^{14}$ is a halogen atom, or a $C_1$ to $C_3$ alkyl, $C_1$ to $C_3$ alkoxy, $CF_3$, $NO_2$, CN or $OCF_2H$ group; and salts of the acidic species with alkali metal bases, ammonia and amines, which process comprises treating an aniline or isocyanate of formula II:

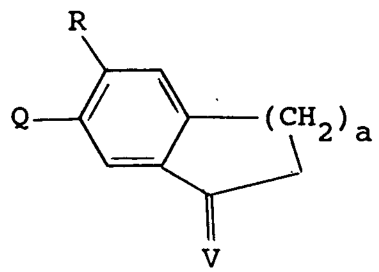

(II)

wherein Q is $-NH_2$ or -NCO with the appropriate reagents to introduce any one of the groups J-1 to J-14 and, optionally, oximating a compound of formula I where V is an oxygen atom to a compound of Formula I where V is the group N-O-$R^1$.

**2.** The process of Claim 1 where R is a hydrogen or halogen atom or a $C_1$ to $C_3$ alkyl group; $R^1$ is a hydrogen atom or a $C_1$ to $C_6$ alkyl, $C_3$ to $C_5$ alkenyl, $C_3$ to $C_5$ alkynyl, $C_3$ to $C_8$ alkoxycarbonylalkyl, or benzyl group; and $R^{14}$ is a fluorine or chlorine atom or a $CH_3$ or $OCH_3$ group.

**3.** The process of Claim 2, where a is one.

**4.** The process of Claim 2, where a is two.

**5.** The process of Claim 2, where $R^2$ is a fluorine or chlorine atom or a $CH_3$ or $OCH_3$ group; m is 0 or 1; R is a hydrogen, fluorine or chlorine atom or a $C_1$ to $C_3$ alkyl group; $R^1$ is a hydrogen atom or a $C_1$ to $C_4$ alkyl, allyl, propargyl, optionally substituted benzyl, or $C_3$ to $C_8$ alkoxycarbonylalkyl group.

**6.** The process of Claim 5, where
m is 0;
V is $=NOR^1$;
X and Y represent oxygen atoms; and
R is a hydrogen, fluorine or chlorine atom or a $CH_3$ group.

**7.** The process of Claim 6, where $R^3$ and $R^4$ form an alicyclic six-membered ring;
R is a hydrogen atom or a $CH_3$ group;
$R^1$ is a hydrogen atom or a $C_1$ to $C_4$ alkyl, allyl, propargyl, optionally substituted benzyl, or $C_3$ to $C_8$ alkoxycarbonylalkyl group.

**8.** The process of Claim 7, where R is a hydrogen or fluorine atom; and $R^3$ and $R^4$ form $-(CH_2)_4$.

**Patentansprüche**
**Patentansprüche für folgende Vertragsstaaten : AT, BE, CH, DE, FR, GB, GR, IT, LI, LU, NL, SE**

**1.** Verbindung mit der Formel:

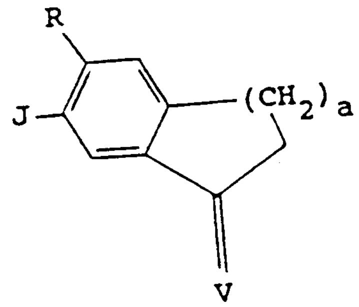

(I),

worin

R ein Wasserstoff- oder Halogenatom; eine Cyanogruppe; eine Alkyl- oder Alkoxygruppe mit 1 bis 4 Kohlenstoffatomen; eine Trihalomethyl- oder Hydroxymethylgruppe; eine Alkyl-, Alkenyl- oder Alkanoyloxymethylgruppe mit bis zu 6 Kohlenstoffatomen; oder eine Gruppe $-OCF_nH_{3-n}$, worin n den Wert 1, 2 oder 3 aufweist, bedeutet;

V ein Sauerstoffatom oder eine Gruppe N-O-R', worin $R^1$ ein Wasserstoffatom; eine Alkyl-, Alkenyl- oder Alkinylgruppe mit bis zu 6 Kohlenstoffatomen, wobei die Alkylgruppe gewünschtenfalls durch Cyano, Mono- oder Polyhalogen oder Hydroxy- oder Alkoxycarbonyl substituiert sein kann und die Alkenylgruppe gewünschtenfalls Mono- oder Polyhalogen-substituiert sein kann; eine Phenylgruppe, die gewünschtenfalls durch eine oder mehrere Gruppen $R^{14}$, wie nachfolgend definiert, substituiert sein kann, oder eine Phenylalkylgruppe mit 7 bis 10 Kohlenstoffatomen, die gewünschtenfalls durch eine oder mehrere Gruppen $R^{14}$ gemäß nachstehender Definition substituiert sein kann; oder eine Heteroaryl- oder Heteroaralkylgruppe bedeutet, worin der Heteroarylrest Furanyl, Pyrrolyl, Thienyl, Pyridinyl, Thiazolyl, Isoxazolyl, Pyrazolyl, Imidazolyl, Triazolyl (1,2,3- oder 1,2,4-), Benzofuranyl, Indolyl, Indazolyl, Thianaphthenyl, Pyridazinyl, Pyrimidinyl, Pyrazinyl, Cinnolinyl, Chinazolinyl oder Chinoxalinyl ist;

a den Wert eins oder zwei hat;

J für einen der folgenden Reste steht:

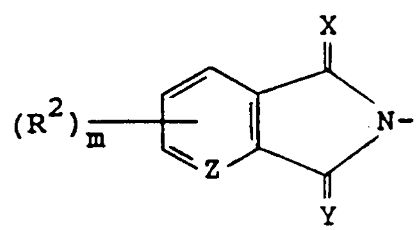

J-1

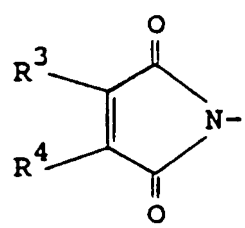

J-2

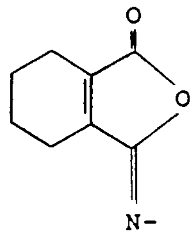

J-3

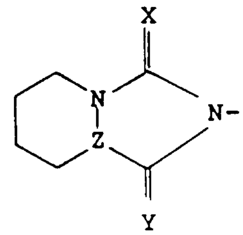

J-4

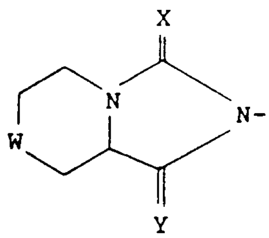

J-5

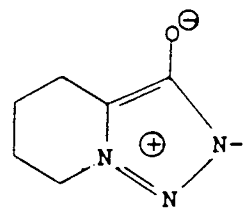

J-6

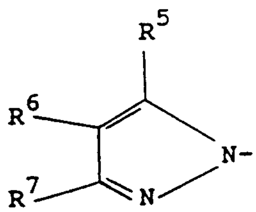

J-7

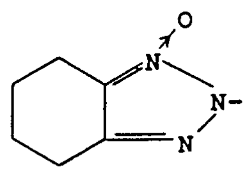

J-8

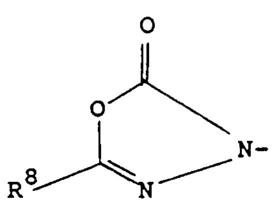

J-9

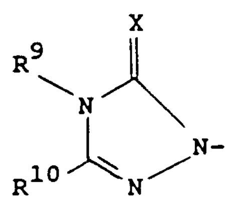

J-10

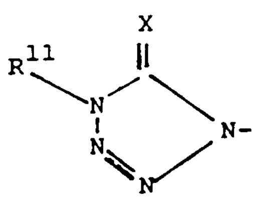

J-11

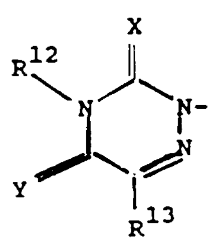

J-12

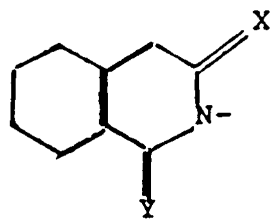

J-13

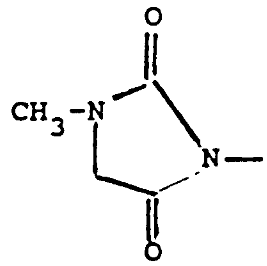

J-14

worin

| | |
|---|---|
| X und Y | jeweils unabhängig voneinander Sauerstoff oder Schwefel bedeuten; |
| Z | für CH oder N steht; |
| W | für S oder $SO_2$ steht; |
| m | den Wert null, eins oder zwei hat; |
| $R^2$ | ein Halogenatom bedeutet oder eine Alkyl-, Haloalkyl- oder Alkoxyalkylgruppe mit 1 bis 4 Kohlenstoffatomen darstellt; |
| $R^3$ und $R^4$ | jeweils unabhängig voneinander ein Wasserstoffatom oder eine Alkylgruppe mit 1 bis 4 Kohlenstoffatomen bedeuten oder $R^3$ und $R^4$ zusammen mit dem Kohlenstoffatom, an das sie gebunden sind, einen alicylischen sechs-gliedrigen Ring bilden, der durch eine bis drei Methylgruppen substituiert sein kann; |
| $R^5$ | ein Wasserstoff- oder Halogenatom oder eine Alkyl- oder Alkyl-$S(O)_b$-Gruppe mit einem bis vier Kohlenstoffatomen bedeuten, worin b den Wert null, eins oder zwei hat; |
| $R^6$ | ein Wasserstoff- oder Halogenatom oder eine Alkyl-, Alkylthio- oder Alkylsulfonylgruppe mit einem bis vier Kohlenstoffatomen darstellt; |
| $R^7$ | eine Alkylgruppe mit einem bis vier Kohlenstoffatomen darstellt oder $R^6$ und $R^7$ zusammen den Rest $-(CH_2)_c-$ ausbilden, worin c den Wert drei oder vier aufweist, welcher Rest durch ein bis drei Methylgruppen substituiert sein kann; |
| $R^8$ | ein Wasserstoffatom oder eine Alkyl-, Alkenyl-, Halogenalkyl- oder Alkoxyalkylgruppe mit bis zu 6 Kohlenstoffatomen oder eine Cycloalkylgruppe mit 3 bis 6 Kohlenstoffatomen, eine Cycloalkylalkylgruppe mit 4 bis 8 Kohlenstoffatomen bedeutet, wobei jede dieser Gruppen durch ein bis drei Alkylgruppen mit ein bis vier Kohlenstoffatomen substituiert sein kann; |
| $R^9$ | eins bis sechs Kohlenstoffatome aufweist und eine Alkyl-, Haloalkyl-, Cyanoalkyl-, Alkoxyalkyl-, Alkenyl-, Alkinyl- oder -Alkyl-$S(O)_a$-Alkyl-Gruppe ist, worin a den Wert null, eins oder zwei hat; |
| $R^{10}$ | ein Wasserstoffatom oder eine Gruppe $R^9$ gemäß vorstehender Definition ist oder $R^9$ und $R^{10}$ zusammen einen Alkylenrest $-(CH_2)_c-$ wie oben für $R^7$ definiert, oder einen Rest $-(CH_2)_d-U-(CH_2)_e-$ darstellen, worin d und e jeweils für null, eins, zwei oder drei stehen und d plus e den Wert zwei oder drei hat und U für Sauerstoff, $-S(O)_a-$ oder -NRAlkyl steht, worin a den Wert null, eins oder zwei hat, mit der Maßgabe, daß dann, wenn U für - $S(O)_a$- steht, d einen anderen Wert als null hat; |
| $R^{11}$ | ein bis sechs Kohlenstoffatome aufweist und eine Alkyl-, Haloalkyl-, Alkoxyalkyl-, Alkylthioalkyl-, Cyanoalkyl-, Haloalkoxyalkyl-, Alkenyl- oder Haloalkenylgruppe ist; |
| $R^{12}$ | ein Wasserstoffatom oder eine Aminogruppe darstellt oder ein bis vier Kohlenstoffatome aufweist und eine Alkyl-, Haloalkyl-, Cyanoalkyl-, Alkenyl-, Alkinyl-, Alkoxyalkyl- oder Alkoxycarbonylgruppe ist; |
| $R^{13}$ | eine Gruppe $R^{11}$ gemäß vorstehender Definition ist oder ein Wasserstoffatom oder eine Carboxyl- oder Alkoxycarbonylgruppe darstellt; |
| $R^{14}$ | ein Halogenatom ist oder eine $C_1$- bis $C_3$-Alkylgruppe, $C_1$- bis $C_3$Alkoxygruppe, $CF_3$-, $NO_2$-, CN- oder $OCF_2H$-Gruppe bezeichnet; |

und Salze der sauren Verbindungen mit Alkalimetallbasen, Amoniak und Aminen.

**2.** Verbindungen nach Anspruch 1, worin

R ein Wasserstoff- oder Halogenatom oder eine $C_1$- bis $C_3$-Alkylgruppe bedeutet;
$R^1$ für ein Wasserstoffatom oder eine $C_1$- bis $C_6$-Alkylgruppe, $C_3$-$C_5$-Alkenylgruppe,$C_3$- bis $C_5$-Alkinylgruppe, $C_3$- bis $C_8$-Alkoxycarbonylalkylgruppe oder eine Benzylgruppe steht; und
$R^{14}$ ein Fluor- oder Chloratom oder eine $CH_3$- oder $OCH_3$-Gruppe bedeutet.

**3.** Verbindungen nach Anspruch 2, worin a den Wert null hat.

**4.** Verbindungen nach Anspruch 2, worin a den Wert zwei hat.

**5.** Verbindungen nach Anspruch 2, worin
$R^2$ ein Fluor- oder Chloratom oder eine $CH_3$- oder $OCH_3$-Gruppe bedeutet;
m den Wert 0 oder 1 hat;
R für ein Wasserstoff-, Fluor- oder Chloratom oder eine eine $C_1$- bis $C_3$-Alkylgruppe steht;
$R^1$ ein Wasserstoffatom oder eine $C_1$- bis $C_4$-Alkylgruppe, Allylgruppe, Propargylgruppe, gegebenenfalls substituierte Benzylgruppe oder $C_3$- bis $C_8$-Alkoxycarbonylalkylgruppe steht.

**6.** Verbindungen nach Anspruch 5, worin
m den Wert 0 hat;
V für $=NOR^1$ steht;
X und Y Sauerstoffatome bedeuten; und
R ein Wasserstoff-, Fluor- oder Chloratom oder eine $CH_3$-Gruppe bedeutet.

**7.** Verbindungen nach Anspruch 6, worin $R^3$ und $R^4$ einen alicyclischen sechs-gliedrigen Ring ausbilden;
R ein Wasserstoffatom oder eine $CH_3$-Gruppe bedeutet;
$R^1$ ein Wasserstoffatom oder eine $C_1$- bis $C_4$-Alkylgruppe, Allylgruppe, Propargylgruppe, gegebenenfalls substituierte Benzylgruppe oder eine $C_3$- bis $C_8$-Alkoxycarbonylalkylgruppe darstellt.

**8.** Verbindungen nach Anspruch 7, worin R ein Wasserstoff- oder Fluoratom bedeutet; und $R^3$ und $R^4$ eine Gruppe $-(CH_2)_4-$ ausbilden.

**9.** Eine Verbindunge mit der Formel

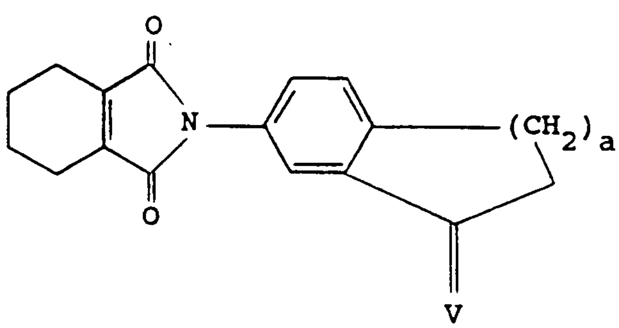

worin V für N-O-R" steht und entweder a den Wert 1 aufweist und R" eine (Ethoxycarbonyl)-1-(1-ethoxycarbonyl)methylgruppe ist oder a den Wert 2 hat und R" eine Tetrahydropyranylgruppe darstellt.

**10.** Verfahren zur Herstellung einer Verbindung der Formel I, wie in einem der vorstehenden Ansprüche definiert, umfassend ein Behandeln eines Anilins oder Isocyanats der Formel II:

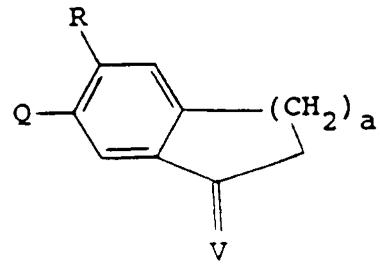

(II),

worin Q für $-NH_2$ oder -NCO steht, mit den entsprechenden Reagenzien zur Einführung einer der Gruppen J-1 bis J-14 und gegebenenfalls ein Oximieren einer Verbindung der Formel I, worin V ein Sauerstoffatom bedeutet, zu einer Verbindung der Formel I, worin V die Gruppe $N-O-R^1$ bedeutet.

**11.** Verbindung nach einem der vorstehenden Ansprüche 1 bis 9, wenn sie nach dem Verfahren von Anspruch 10 hergestellt ist.

**12.** Zur Bekämpfung des Wachstums von unerwünschter Vegetation geeignete Zusammesetzung, die eine wirksame Menge einer Verbindung, wie in einem der Ansprüche 1 bis 9 oder 11 beansprucht, und wenigstens ein grenzflächenaktives Mittel, ein festes oder ein flüssiges Verdünnungsmittel umfaßt.

**13.** Verfahren zur Bekämpfung des Wachstums von unerwünschter Vegetation, welches ein Aufbringen einer wirksamen Menge einer Verbindung, wie in einem der Ansprüche 1 bis 9 oder 11 beansprucht, oder einer Zusammensetzung, wie in Anspruch 12 beansprucht, auf den zu schützenden Ort umfaßt.

**14.** Verwendung einer Verbindung, wie in einem der Ansprüche 1 bis 9 oder 11 beansprucht, als Herbizide.

**Patentansprüche für folgenden Vertragsstaat : ES**

**1.** Verfahren zur Herstellung einer Verbindung mit der Formel:

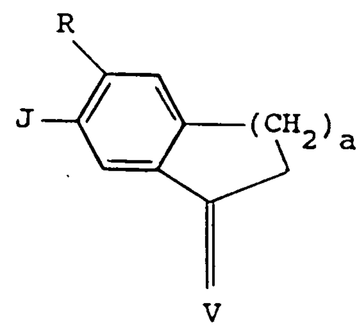

(I),

worin

R ein Wasserstoff- oder Halogenatom; eine Cyanogruppe; eine Alkyl- oder Alkoxygruppe mit 1 bis 4 Kohlenstoffatomen; eine Trihalomethyl- oder Hydroxymethylgruppe; eine Alkyl-, Alkenyl- oder Alkanoyloxymethylgruppe mit bis zu 6 Kohlenstoffatomen; oder eine Gruppe $-OCF_nH_{3-n}$, worin n den Wert 1, 2 oder 3 aufweist, bedeutet;

V ein Sauerstoffatom oder eine Gruppe $N-O-R^1$, worin $R^1$ ein Wasserstoffatom; eine Alkyl-, Alkenyl- oder Alkinylgruppe mit bis zu 6 Kohlenstoffatomen, wobei die Alkylgruppe gewünschtenfalls durch Cyano, Mono- oder Polyhalogen oder Hydroxy- oder Alkoxycarbonyl substituiert sein kann und die Alkenylgruppe gewünschtenfalls Mono- oder Polyhalogen-substituiert sein kann; eine Phenylgruppe, die gewünschtenfalls durch eine oder mehrere Gruppen $R^{14}$, wie nachfolgend definiert, substituiert sein kann, oder eine Phenylalkylgruppe mit 7 bis 10 Kohlenstoffatomen, die gewünschtenfalls durch eine oder mehrere Gruppen $R^{14}$ gemäß nachstehender Definition substituiert sein kann; oder eine Heteroaryl- oder Heteroaralkylgruppe bedeutet, worin der Heteroarylrest Furanyl, Pyrrolyl, Thienyl, Pyridinyl, Thiazolyl, Isoxazolyl,

Pyrazolyl, Imidazolyl, Triazolyl (1,2,3- oder 1,2,4-), Benzofuranyl, Indolyl, Indazolyl, Thianaphthenyl, Pyridazinyl, Pyrimidinyl, Pyrazinyl, Cinnolinyl, Chinazolinyl oder Chinoxalinyl ist;

a den Wert eins oder zwei hat;

J für einen der folgenden Reste steht:

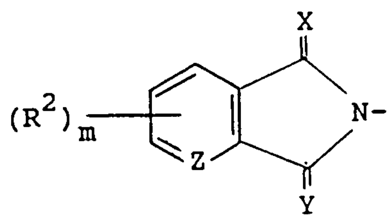

J-1

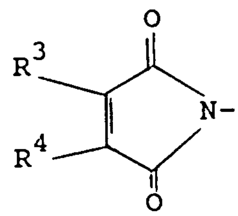

J-2

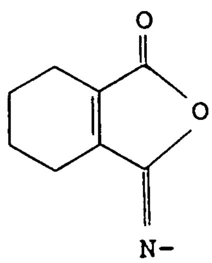

J-3

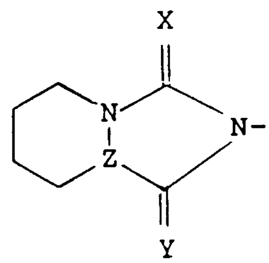

J-4

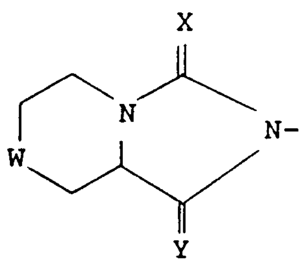

J-5

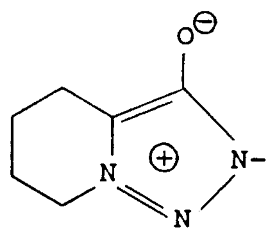

J-6

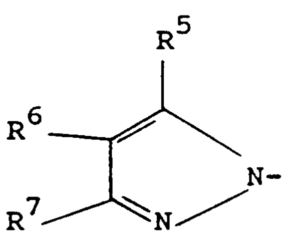

J-7

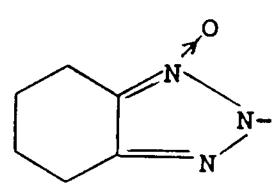

J-8

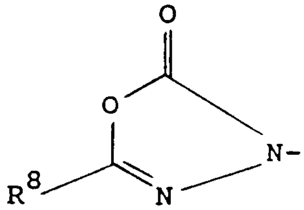

J-9

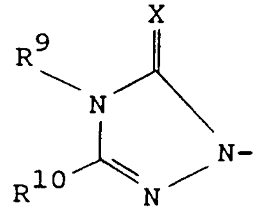

J-10

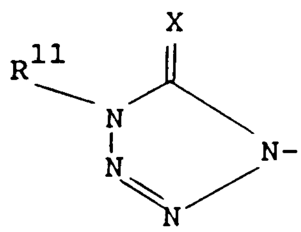

J-11

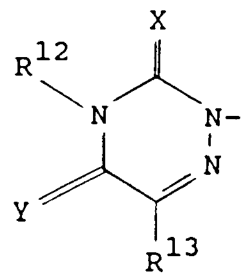

J-12

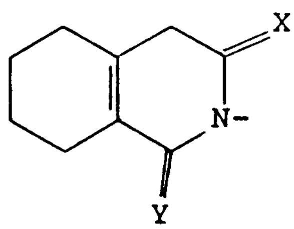

J-13

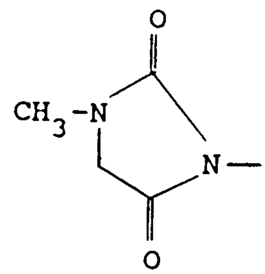

J-14

worin

- X und Y — jeweils unabhängig voneinander Sauerstoff oder Schwefel bedeuten;
- Z — für CH oder N steht;
- W — für S oder $SO_2$ steht;
- m — den Wert null, eins oder zwei hat;
- $R^2$ — ein Halogenatom bedeutet oder eine Alkyl-, Haloalkyl- oder Alkoxyalkylgruppe mit 1 bis 4 Kohlenstoffatomen darstellt;
- $R^3$ und $R^4$ — jeweils unabhängig voneinander ein Wasserstoffatom oder eine Alkylgruppe mit 1 bis 4 Kohlenstoffatomen bedeuten oder $R^3$ und $R^4$ zusammen mit dem Kohlenstoffatom, an das sie gebunden sind, einen alicylischen sechs-gliedrigen Ring bilden, der durch eine bis drei Methylgruppen substituiert sein kann;
- $R^5$ — ein Wasserstoff- oder Halogenatom oder eine Alkyl- oder Alkyl-$S(O)_b$-Gruppe mit einem bis vier Kohlenstoffatomen bedeuten, worin b den Wert null, eins oder zwei hat;
- $R^6$ — ein Wasserstoff- oder Halogenatom oder eine Alkyl-, Alkylthio- oder Alkylsulfonylgruppe mit einem bis vier Kohlenstoffatomen darstellt;
- $R^7$ — eine Alkylgruppe mit einem bis vier Kohlenstoffatomen darstellt oder $R^6$ und $R^7$ zusammen den Rest $-(CH_2)_c-$ ausbilden, worin c den Wert drei oder vier aufweist, welcher Rest durch ein bis drei Methylgruppen substituiert sein kann;
- $R^8$ — ein Wasserstoffatom oder eine Alkyl-, Alkenyl-, Halogenalkyl- oder Alkoxyalkylgruppe mit bis zu 6 Kohlenstoffatomen oder eine Cycloalkylgruppe mit 3 bis 6 Kohlenstoffatomen, eine Cycloalkylalkylgruppe mit 4 bis 8 Kohlenstoffatomen bedeutet, wobei jede dieser Gruppen durch ein bis drei Alkylgruppen mit ein bis vier Kohlenstoffatomen substituiert sein kann;
- $R^9$ — eins bis sechs Kohlenstoffatome aufweist und eine Alkyl-, Haloalkyl-, Cyanoalkyl-, Alkoxyalkyl-, Alkenyl-, Alkinyl- oder -Alkyl-$S(O)_a$-Alkyl-Gruppe ist, worin a den Wert null, eins oder zwei hat;
- $R^{10}$ — ein Wasserstoffatom oder eine Gruppe $R^9$ gemäß vorstehender Definition ist oder $R^9$ und $R^{10}$ zusammen einen Alkylenrest $-(CH_2)_c-$ wie oben für $R^7$ definiert, oder einen Rest $-(CH_2)_d-U-(CH_2)_e-$ darstellen, worin d und e jeweils für null, eins, zwei oder drei stehen und d plus e den Wert zwei oder drei hat und U für Sauerstoff, $-S(O)_a-$ oder -NRAlkyl steht, worin a den Wert null, eins oder zwei hat, mit der Maßgabe, daß dann, wenn U für - $S(O)_a$- steht, d einen anderen Wert als null hat;

$R^{11}$ ein bis sechs Kohlenstoffatome aufweist und eine Alkyl-, Haloalkyl-, Alkoxyalkyl-, Alkylthioalkyl-, Cyanoalkyl-, Haloalkoxyalkyl-, Alkenyl- oder Haloalkenylgruppe ist;

$R^{12}$ ein Wasserstoffatom oder eine Aminogruppe darstellt oder ein bis vier Kohlenstoffatome aufweist und eine Alkyl-, Haloalkyl-, Cyanoalkyl-, Alkenyl-, Alkinyl-, Alkoxyalkyl- oder Alkoxycarbonylgruppe ist;

$R^{13}$ eine Gruppe $R^{11}$ gemäß vorstehender Definition ist oder ein Wasserstoffatom oder eine Carboxyl- oder Alkoxycarbonylgruppe darstellt;

$R^{14}$ ein Halogenatom ist oder eine $C_1$- bis $C_3$-Alkylgruppe, $C_1$- bis $C_3$Alkoxygruppe, $CF_3$-, $NO_2$-, CN- oder $OCF_2H$-Gruppe bezeichnet; und von Salzen der sauren Verbindungen mit Alkalimetallbasen, Ammoniak und Aminen, welches Verfahren ein Behandeln eines Anilins oder Isocyanats der Formel II

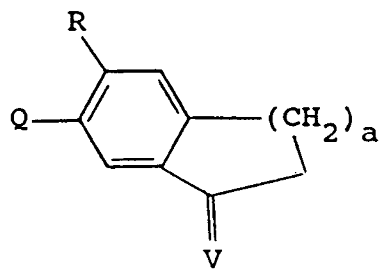

(II),

worin Q für $-NH_2$ oder -NCO steht, mit den entsprechenden Reagenzien zur Einführung einer der Gruppen J-1 bis J-14 und gewünschtenfalls ein Oximieren einer Verbindung der Formel I, worin V ein Sauerstoffatom bedeutet, zu einer Verbindung der Formel I, worin V die Gruppe N-O-$R^1$ darstellt, umfaßt.

**2.** Verfahren nach Anspruch 1, worin

R ein Wasserstoff- oder Halogenatom oder eine $C_1$- bis $C_3$-Alkylgruppe bedeutet;

$R^1$ für ein Wasserstoffatom oder eine $C_1$- bis $C_6$-Alkylgruppe, $C_3$-$C_5$-Alkenylgruppe,$C_3$- bis $C_5$-Alkinylgruppe, $C_3$- bis $C_8$-Alkoxycarbonylalkylgruppe oder eine Benzylgruppe steht; und

$R^{14}$ ein Fluor- oder Chloratom oder eine $CH_3$- oder $OCH_3$-Gruppe bedeutet.

**3.** Verfahren nach Anspruch 2, worin a den Wert 0 hat.

**4.** Verfahren nach Anspruch 2, worin a den Wert zwei hat.

**5.** Verfahren nach Anspruch 2, worin

$R^2$ ein Fluor- oder Chloratom oder eine $CH_3$- oder $OCH_3$-Gruppe bedeutet;

m den Wert 0 oder 1 hat;

R für ein Wasserstoff-, Fluor- oder Chloratom oder eine $C_1$- bis $C_3$-Alkylgruppe steht;

$R^1$ ein Wasserstoffatom oder eine $C_1$- bis $C_4$-Alkylgruppe, Allylgruppe, Propargylgruppe, gegebenenfalls substituierte Benzylgruppe oder $C_3$- bis $C_8$-Alkoxycarbonylalkylgruppe steht.

**6.** Verfahren nach Anspruch 5, worin

m den Wert 0 hat;

V für =NOR$^1$ steht;

X und Y Sauerstoffatome bedeuten; und

R ein Wasserstoff-, Fluor- oder Chloratom oder eine $CH_3$-Gruppe bedeutet.

**7.** Verfahren nach Anspruch 6, worin $R^3$ und $R^4$ einen alicyclischen sechs-gliedrigen Ring ausbilden;

R ein Wasserstoffatom oder eine $CH_3$-Gruppe bedeutet;

$R^1$ ein Wasserstoffatom oder eine $C_1$- bis $C_4$-Alkylgruppe, Allylgruppe, Propargylgruppe, gegebenenfalls substituierte Benzylgruppe oder eine $C_3$- bis bis $C_8$-Alkoxycarbonylalkylgruppe darstellt.

8. Verfahren nach Anspruch 7, worin R ein Wasserstoff- oder Fluoratom bedeutet; und $R^3$ und $R^4$ eine Gruppe $-(CH_2)_4-$ ausbilden.

**Revendications**

**Revendications pour les Etats contractants suivants : AT, BE, CH, DE, FR, GB, GR, IT, LI, LU, NL, SE**

1. Un composé de la formule:

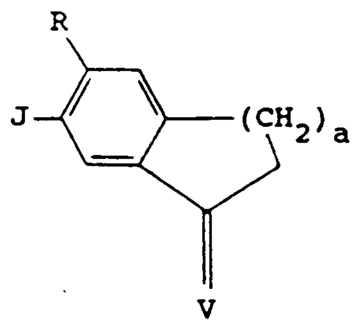

(I)

dans laquelle:

R est un atome d'hydrogène ou d'halogène; un groupe cyano; un groupe alkyle ou alkoxy présentant de 1 à 4 atomes de carbone; un groupe trihalométhyle ou hydroxyméthyle; un groupe alkyl-, alcényl-, ou alkanoyl-oxyméthyle ayant jusqu'à six atomes de carbone; ou un groupe $-OCF_nH_{3-n}$ dans laquelle n = un, deux ou trois;

V est un atome d'hydroxygène ou un groupe N-O-$R^1$, dans lequel $R^1$ représente un atome d'hydrogène; un groupe alkyle, alcényle ou alcynyle présentant jusqu'à six atomes de carbone, le groupe alkyle étant, le cas échéant substitué par un groupe cyano, mono-ou poly-halo ou bien hydroxy- ou alkoxy-carbonyle et le groupe alcényle, étant le cas échéant, substitué par un groupe mono- ou poly-halo; un groupe phényle substitué, le cas échéant, par un ou plus d'un groupe $R^{14}$ défini ci-dessous ou un groupe phényl-alkyle présentant de sept à dix atomes de carbone, le cas échéant, substitué par un ou plus d'un groupe $R^{14}$, tel que défini ci-dessous; ou un groupe hétéroaryle ou hétéroaralkyle dans lequel la partie hétéroaryle est un groupe furanyle, pyrrolyle, thiényle, pyridinyle, thiazolyle, isoxazolyle, pyrazolyle, imidazolyle, (1,2,3-ou 1,2,4-) triazolyle, benzofuranyle, indolyle, indazolyle, thianaphthényle, pyridazinyle, pyrimidinyle, pyrazinyle, cinnolinyle, quinazolinyle ou quinoxalinyle;

a est un ou deux;

J est l'une des parties :

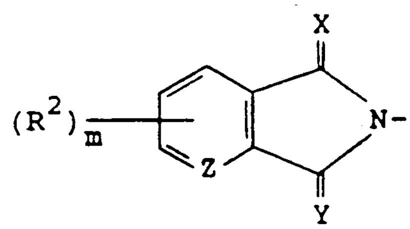

J-1

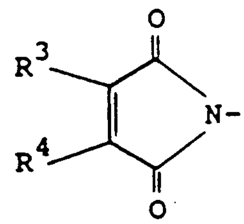

J-2

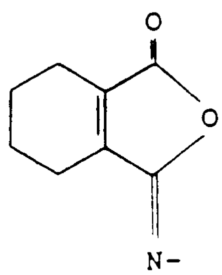

J-3

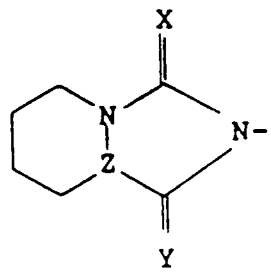

J-4

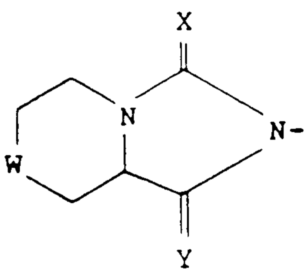

J-5

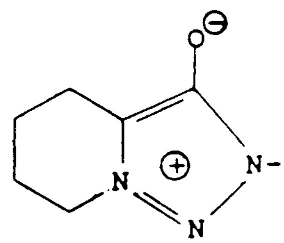

J-6

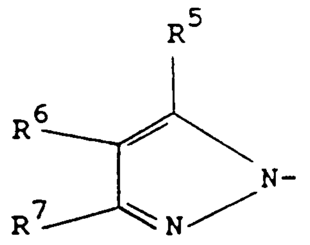

J-7

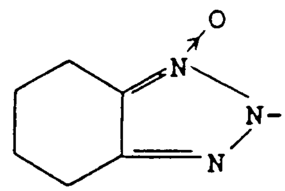

J-8

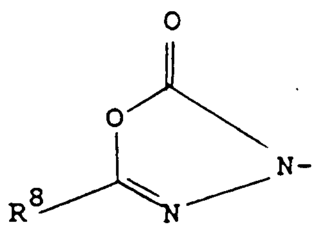

J-9

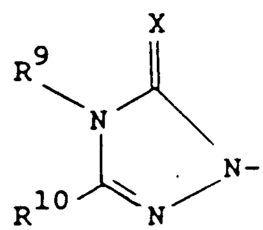

J-10

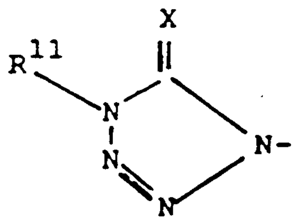

J-11

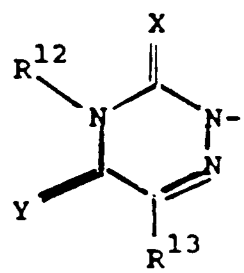

J-12

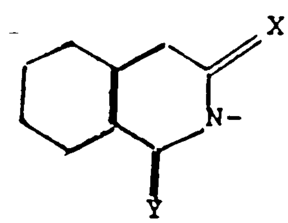

J-13

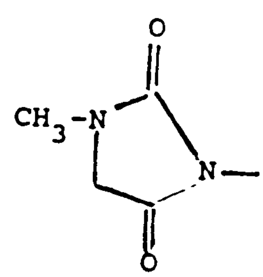

J-14

dans lesquelles

X et Y représentent chacun, indépendamment, O ou S;

Z est CH ou N;

W est S ou $SO_2$;

m est zéro, un ou deux;

$R^2$ est un atome d'halogène, ou est un groupe alkyle, haloalkyle ou alkoxy possédant de un à quatre atomes de carbone;

$R^3$ et $R^4$ représentent chacun, indépendamment, un atome d'hydrogène ou un groupe alkyle possédant de un a quatre atomes de carbone, ou bien $R^3$ et $R^4$ conjointement avec les atomes de carbone auxquels ils sont attachés forment un cycle alicyclique à six éléments, qui peut être substitué par de un à trois groupes méthyle;

$R^5$ est un atome d'hydrogène, ou un atome d'halogène, ou bien un groupe alkyle ou alkyl-$S(O)_b$ possédant de un à quatre atomes de carbone, dans lequel b est zéro, un ou deux;

$R^6$ est un atome d'hydrogène ou d'halogène, ou bien un groupe alkyle, alkylthio ou alkylsulfonyle possédant de un à quatre atomes de carbone;

$R^7$ est un groupe alkyle possédant de un à quatre atomes de carbone, ou bien $R^6$ et $R^7$ forment conjointement la partie $-(CH_2)_c-$, dans laquelle c est trois ou quatre, qui peut être substituée par un, deux ou trois groupes méthyle;

$R^8$ est un atome d'hydrogène ou bien un groupe alkyle, alcényle, haloalkyle ou alkoxyalkyle présentant jusqu'à six atomes de carbone, ou bien un groupe cycloalkyle possédant de trois à six atomes de carbone, un groupe cycloalkylalkyle possédant de quatre à huit atomes de carbone, ou bien l'un quelconque de ces groupes substitués par de un à trois groupes alkyle, présentant chacun de un à quatre atomes de carbone,

$R^9$ possède de un à six atomes de carbone et est un groupe alkyle, haloalkyle, cyanoalkyle, alkoxyalkyle, alcényle, alkynyle, ou -alkyl-$S(O)_a$-alkyle dans lequel a est zéro, un ou deux;

$R^{10}$ est un atome d'hydrogène, un groupe $R^9$ tel que défini ci-dessus, ou bien $R^9$ et $R^{10}$ représentent ensemble une partie alcylène $-(CH_2)_c-$, comme défini ci-dessus pour $R^7$, ou bien une partie $-(CH_2)_d-U-(CH_2)_e-$ dans lequel d et e représentent chacun zéro, un, deux ou trois, et d plus e = deux ou trois, et U est de l'oxygene, $-S(O)_a-$ ou -NRalkyle où a est zéro, un ou deux sous la condition que lorsque U est $-S(O)_a-$, d est différent de zéro;

$R^{11}$ possède de un à six atomes de carbone, et est un groupe alkyle, haloalkyle, alkoxyalkyle, alkylthioalkyle, cyanoalkyle, haloalkoxyalkyle, alcényle ou haloalcényle;

$R^{12}$ est un atome d'hydrogène ou un groupe amino, ou bien possède dé un à quatre atomes de carbone et est un groupe alkyle, haloalkyle, cyanoalkyle, alcényle, alkynyle, alkoxyalkyle ou alkoxycarbonyle;

$R^{13}$ est un groupe $R^{11}$ tel que défini ci-dessus, ou bien est un atome d'hydrogène, ou un groupe carboxyle ou alkoxycarbonyle;

$R^{14}$ est un atome d'halogène ou bien un groupe alkyle en $C_1$ à $C_3$, alkoxy en $C_1$ à $C_3$, $CF_3$, $NO_2$, CN ou $OCF_2H$;

ainsi que les sels des espèces acides avec des bases de métaux alcalins, l'ammoniac et des amines.

**2.** Composés selon la revendication 1, dans lesquels

R est un atome d'hydrogène ou d'halogène ou un groupe alkyle en $C_1$ à $C_3$, $R^1$ est un atome d'hydrogène où un groupe alkyle en $C_1$ à $C_6$, alcényle en $C_3$ à $C_5$, alkynyle en $C_3$ à $C_5$, alkoxycarbonylalkyle en $C_3$ à $C_8$, ou bien un groupe benzyle; et $R^{14}$ est un atome de fluor ou de chlore ou bien un groupe $CH_3$ ou $OCH_3$.

**3.** Composés selon la revendication 2, dans lesquels a est un.

**4.** Composés selon la revendication 2, dans lesquels a est deux.

**5.** Composés selon la revendication 2, dans lesquels $R^2$ est un atome de fluor ou de chrome ou bien un groupe $CH_3$ ou $OCH_3$; m est 0 ou 1.

R est un atome d'hydrogène, de fluor ou de chlore, ou bien un groupe alkyle en $C_1$ à $C_3$; $R^1$ est un atome d'hydrogène ou un groupe alkyle en $C_1$ à $C_4$, allyle, propargyle, substitué, le cas échéant, par du benzyle, ou bien un groupe alkoxycarbonylalkyle en $C_3$ à $C_8$.

**6.** Composés selon la revendication 5, dans lesquels
m est 0;
V est =$NOR^1$;
X et Y représentent des atomes d'oxygène ; et
R est un atome d'hydrogène, de fluor ou de chlore ou bien un groupe $CH_3$.

**7.** Composés selon la revendication 6, dans lesquels
$R^3$ et $R^4$ forment un noyau alicyclique à 6 éléments;
R est un atome d'hydrogène ou un groupe $CH_3$;
$R^1$ est un atome d'hydrogène ou un groupe alkyle en $C_1$ à $C_4$, allyle, propargyle, substitué, le cas échéant, par du benzyle, ou bien un groupe alkoxycarbonylalkyle en $C_3$ à $C_8$.

**8.** Composés de la revendication 7, dans lesquels R est un atome d'hydrogène de fluor, et $R^3$ et $R^4$ forment $(CH_2)_4$.

**9.** Un composé de la Formule

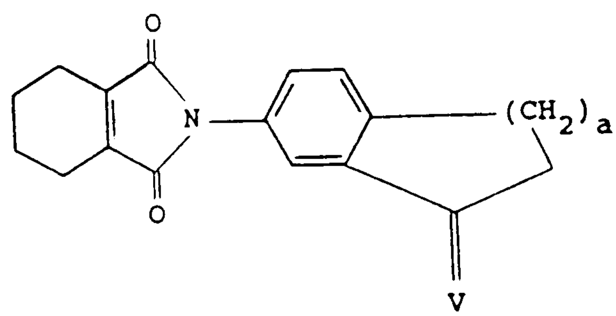

dans laquelle Y est -N-O-R", et soit a est 1 et R" est un groupe (éthoxycarbonyl)-1-(1-éthoxycarbonyl) méthyle soit a est 2 et R" est un groupe tétrahydropyranyle.

**10.** Un procédé pour la préparation d'un composé de la Formule I tel que défini dans l'une quelconque des revendications précédentes, procédé dans lequel on traite une aniline du un isocyanate de Formule II:

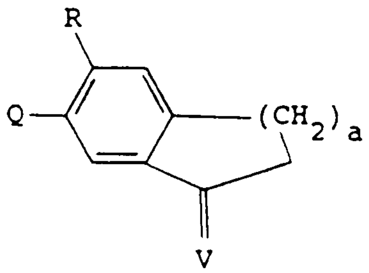

(II)

dans laquelle Q est $-NH_2$ ou -NCO avec les réactifs appropriés afin d'introduire l'un quelconque des groupes J-1 à J-14 et, le cas échéant, à oximer un composé de Formule I dans laquelle V est un atome d'oxygène en un composé de formule I dans laquelle V est le groupe $N-O-R^1$.

**11.** Un composé tel que défini dans l'une quelconque des revendications précédentes de 1 à 9, lorsqu'il est préparé par le procédé de la revendication 10.

**12.** Une composition appropriée pour contrôler la croissance d'une végétation indésirable, qui, comprend une quantité efficace d'un composé tel que revendiqué dans l'une quelconque des revendications 1 à 9 ou 11 et au moins un diluant tensioactif, solide ou liquide.

**13.** Une méthode pour contrôler le développement d'une gestation indésirable, qui consiste à appliquer a l'endroit devant être protégé une quantité efficace d'un composé tel que revendiqué dans l'une quelconque des revendications 1 à 9 ou 11 ou bien une composition telle que revendiquée dans la

revendication 12.

**14.** L'utilisation d'un composé tel que revendiqué dans l'une quelconque des revendications 1 à 9 ou 11 en tant qu'herbicide.

**Revendications pour l'Etat contractant suivant : ES**

**1.** Un procédé pour la préparation d'un composé de la formule:

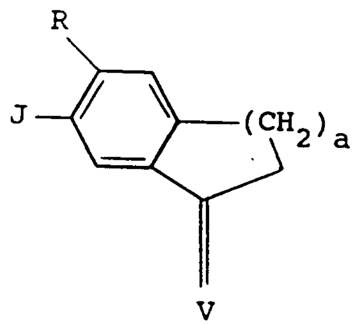

(I)

dans laquelle:

R est un atome d'hydrogène ou d'halogène; un groupe cyano; un groupe alkyle ou alkoxy présentant de 1 à 4 atomes de carbone; un groupe trihalométhyle ou hydroxyméthyle; un groupe alkyl-, alcényl-, alkanoyl-oxyméthyle ou ayant jusqu'à six atomes de carbone; ou un groupe $-OCF_nH_{3-n}$ dans laquelle n = un, deux ou trois;

V est un atome d'hydroxygène ou un groupe $N-O-R^1$, dans lequel $R^1$ représente un atome d'hydrogène; un groupe alkyle, alcényle ou alcynyle présentant jusqu'à six atomes de carbone, le groupe alkyle étant, le cas échéant substitué par un groupe cyano, mono-ou poly-halo ou bien hydroxy-ou alkoxy-carbonyle et le groupe alcényle, étant le cas échéant, substitué par un groupe mono- ou poly-halo; un groupe phényle substitué, le cas échéant, par un ou plus d'un groupe $R^{14}$ défini ci-dessous ou un groupe phényl-alkyle présentant de sept à dix atomes de carbone, le cas échéant, substitué par un ou plus d'un groupe $R^{14}$, tel que défini ci-dessous; ou un groupe hétéroaryle ou hétéroaralkyle dans lequel la partie hétéroaryle est un groupe furanyle, pyrrolyle, thiényle, pyridinyle, thiazolyle, isoxazolyle, pyrazolyle, imidazolyle, (1,2,3-ou 1,2,4-) triazolyle, benzofuranyle, indolyle, indazolyle, thianaphthényle, pyridazinyle, pyrimidinyle, pyrazinyle, cinnolinyle, quinazolinyle ou quinoxalinyle;

a est un ou deux;

J est l'une des parties :

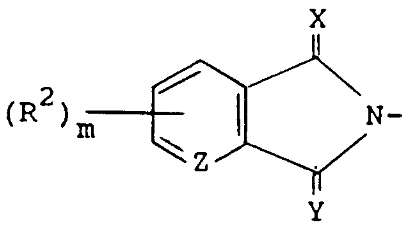

J-1

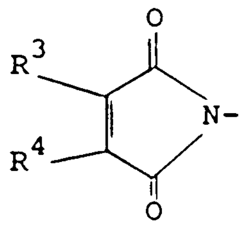

J-2

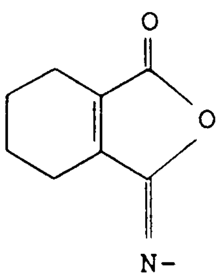

J-3

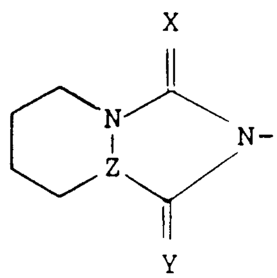

J-4

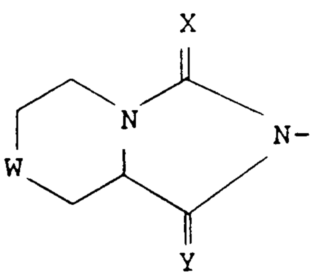

J-5

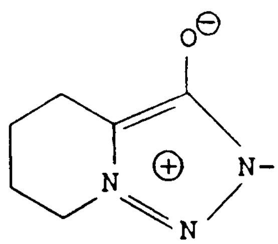

J-6

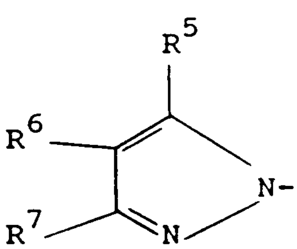

J-7

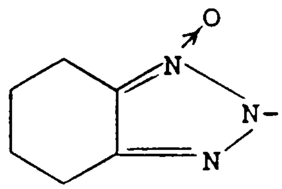

J-8

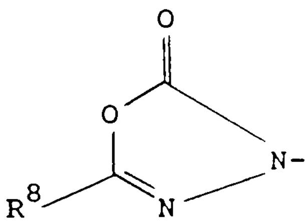

J-9

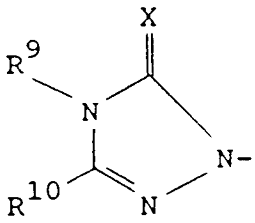

J-10

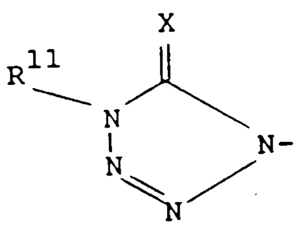

J-11

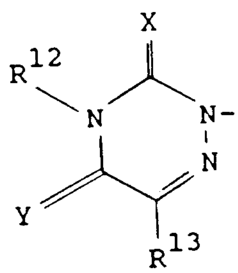

J-12

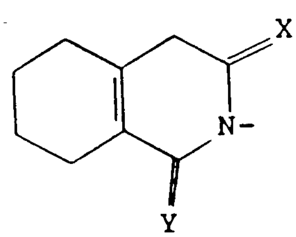

J-13

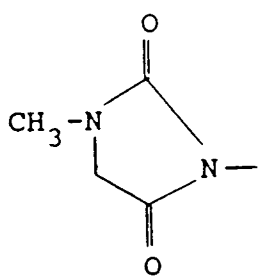

J-14

dans lesquelles

X et Y représentent chacun, indépendamment, O ou S;

Z est CH ou N;

W est S ou $SO_2$;

m est zéro, un ou deux;

$R^2$ est un atome d'halogène, ou est un groupe alkyle, haloalkyle ou alkoxy possédant de un à quatre atomes de carbone;

$R^3$ et $R^4$ représentent chacun, indépendamment, un atome d'hydrogène ou un groupe alkyle possédant de un a quatre atomes de carbone, ou bien $R^3$ et $R^4$ conjointement avec les atomes de carbone auxquels ils sont attachés forment un cycle alicyclique a six éléments, qui peut être substitué

par de un à trois groupes méthyle;

$R^5$ est un atome d'hydrogène, ou un atome d'halogène, ou bien un groupe alkyle ou alkyl-$S(O)_b$ possédant de un à quatre atomes de carbone,

dans lequel b est zéro, un ou deux;

$R^6$ est un atome d'hydrogène ou d'halogène, ou bien un groupe alkyle, alkylthio ou alkylsulfonyle possédant de un à quatre atomes de carbone;

$R^7$ est un groupe alkyle possédant de un à quatre atomes de carbone, ou bien $R^6$ et $R^7$ forment conjointement la partie $-(CH_2)_c-$, dans laquelle c est trois ou quatre, qui peut être substituée par un, deux ou trois groupes méthyle;

$R^8$ est un atome d'hydrogène ou bien un groupe alkyle, alcényle, haloalkyle ou alkoxyalkyle présentant jusqu'à six atomes de carbone, ou bien un groupe cycloalkyle possédant de trois à six atomes de carbone, un groupe cycloalkylalkyle possédant de quatre à huit atomes de carbone, ou bien l'un quelconque de ces groupes substitués par de un a trois groupes alkyle, présentant chacun de un à quatre atomes de carbone,

$R^9$ possède de un à six atomes de carbone et est un groupe alkyle, haloalkyle, cyanoalkyle, alkoxyalkyle, alcényle, alkynyle, ou -alkyl-$S(O)_a$-alkyle dans lequel a est zéro, un ou deux;

$R^{10}$ est un atome d'hydrogène, un groupe $R^9$ tel que défini ci-dessus, ou bien $R^9$ et $R^{10}$ représentent ensemble une partie alcylène $-(CH_2)_c-$, comme défini ci-dessus pour $R^7$, ou bien une partie $-(CH_2)_d-U-(CH_2)_e-$ dans lequel d et e représentent chacun zéro, un, deux ou trois, et d plus e = deux ou trois, et U est de l'oxygène, $-S(O)_a-$ ou -NRalkyle où a est zéro, un ou deux sous la condition que lorsque U est $-S(O)_a-$, d est différent de zéro;

$R^{11}$ possède de un a six atomes de carbone, et est un groupe alkyle, haloalkyle, alkoxyalkyle, alkylthioalkyle, cyanoalkyle, haloalkoxyalkyle, alcényle ou haloalcényle;

$R^{12}$ est un atome d'hydrogène ou un groupe amino, ou bien possède de un à quatre atomes de carbone et est un groupe alkyle, haloalkyle, cyanoalkyle, alcényle, alkynyle, alkoxyalkyle ou alkoxycarbonyle;

$R^{13}$ est un groupe $R^{11}$ tel que défini ci-dessus, ou bien est un atome d'hydrogène, ou un groupe carboxyle ou alkoxycarbonyle;

$R^{14}$ est un atome d'halogène ou bien un groupe alkyle en $C_1$ à $C_3$, alkoxy en $C_1$ à $C_3$, $CF_3$, $NO_2$, CN ou $OCF_2H$;

ainsi que les sels des espèces acides avec des bases de métaux alcalins, l'ammoniac et des amines, procédé dans lequel on traite une aniline ou un isocyanate de Formule II:

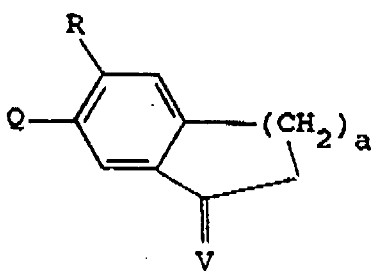

(II)

dans laquelle Q est $-NH_2$ ou -NCO avec les réactifs appropriés afin d'introduire l'un quelconque des groupes J-1 à J-14 et, le cas échéant, à oximer un composé de Formule I dans laquelle V est un atome d'oxygène en un composé de formule I dans laquelle V est le groupe N-O-$R^1$.

2. Le procédé de la revendication 1 dans lequel

R est un atome d'hydrogène ou d'halogène ou un groupe alkyle en $C_1$ à $C_3$, $R^1$ est un atome d'hydrogène ou un groupe alkyle en $C_1$ à $C_6$, alcényle en $C_3$ à $C_5$, alkynyle en $C_3$ à $C_5$, alkoxycarbonylalkyle en $C_3$ à $C_8$, ou bien un groupe benzyle; et $R^{14}$ est un atome de fluor ou de chlore ou bien un groupe $CH_3$ ou $OCH_3$.

3. Le procédé de la revendication 2, dans lequel a est un.

4. Le procédé de la revendication 2, dans lequel a est 2.

5. Le procédé de la revendication 2, dans lequel $R^2$ est un atome de fluor ou de chrome ou bien un

groupe $CH_3$ ou $OCH_3$; m est 0 ou 1.

R est un atome d'hydrogène, de fluor ou de chlore, ou bien un groupe alkyle en $C_1$ à $C_3$; $R^1$ est un atome d'hydrogène ou un groupe alkyle en $C_1$ à $C_4$, allyle, propargyle, substitué, le cas échéant, par du benzyle, ou bien un groupe alkoxycarbonylalkyle en $C_3$ à $C_8$.

**6.** Le procédé de la revendication 5, dans lesquel
m est 0;
V est $=NOR^1$;
X et Y représentent des atomes d'oxygène ; et
R est un atome d'hydrogène, de fluor ou de chlore ou bien un groupe $CH_3$.

**7.** Le procédé de la revendication 6, dans lesquel $R^3$ et $R^4$ forment un noyau alicyclique à 6 éléments;
R est un atome d'hydrogène ou un groupe $CH_3$;
$R^1$ est un atome d'hydrogène ou un groupe alkyle en $C_1$ à $C_4$, allyle, propargyle, substitué, le cas échéant, par du benzyle, ou bien un groupe alkoxycarbonylalkyle en $C_3$ à $C_8$.

**8.** Le procédé de la revendication 7, dans lesquel R est un atome d'hydrogène de fluor, et $R^3$ et $R^4$ forment $(CH_2)_4$.